# EUROPÄISCHE PATENTANMELDUNG

(11) **EP 0 388 353 A1**
(43) Veröffentlichungstag der Anmeldung: **19.09.1990**
(21) Anmeldenummer: 90810128.0
(22) Anmeldetag: 21.02.1990
(51) Int. Cl.: C09B 29/44, C07D 215/38, D06P 1/39

(54) **Azofarbstoffe, deren Herstellung und Verwendung**

(30) Priorität: 06.03.1989 CH 816/89; 24.07.1989 CH 2764/89
(71) Anmelder: CIBA-GEIGY AG, 4002 Basel (CH)
(72) Erfinder: Walter, Harald, Dr., CH-4055 Basel (CH)

(57) **Zusammenfassung**

Azofarbstoffe der Formel 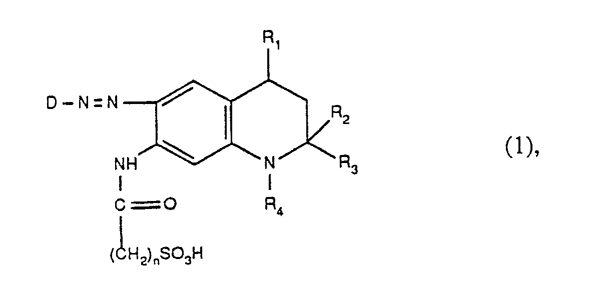 worin D eine Diazokomponente, R₁, R₂, R₃ und R₄ unabhängig voneinander Wasserstoff oder gegebenenfalls substituiertes C₁-C₈-Alkyl und n die Zahl 1, 2, 3, 4 oder 5 ist, ergeben auf stickstoffhaltigen oder hydroxylgruppenhaltigen Fasermaterialien Färbungen von guten Echtheiten.

## Beschreibung

Die vorliegende Erfindung betrifft neue Azofarbstoffe, Verfahren zu deren Herstellung und Verwendung dieser Farbstoffe zum Färben und Bedrucken von Fasermaterialien, insbesondere textilen Fasermaterialien.

Gegenstand der vorliegenden Erfindung sind Azofarbstoffe der Formel 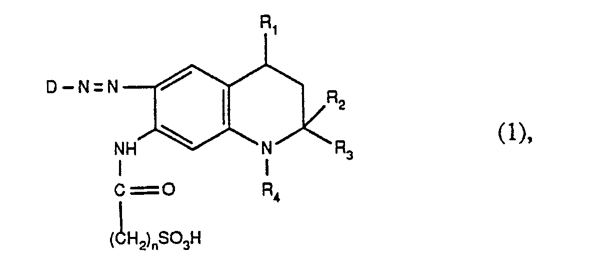 worin D eine Diazokomponente, R₁, R₂, R₃ und R₄ unabhängig voneinander Wasserstoff oder gegebenenfalls substituiertes C₁-C₈-Alkyl und n die Zahl 1,2,3,4 oder 5 ist.

Der Rest D kann die bei Diazokomponenten üblichen Substituenten enthalten, z.B. Alkylgruppen mit 1 bis 8, vorzugsweise 1 bis 4 Kohlenstoffatomen, wie z.B. Methyl, Aethyl, Propyl, Isopropyl, Butyl, Isobutyl, sek.-Butyl, tert.-Butyl, Hexyl, Octyl, wobei die Alkylgruppen durch Sulfo oder Sulfato substituiert sein können, Alkoxygruppen mit 1 bis 8 Kohlenstoffatomen, insbesondere 1 bis 4 Kohlenstoffatomen, wie z.B. Methoxy, Aethoxy, Propoxy, Isopropoxy, Butoxy, Acylaminogruppen wie Alkanoylaminogruppen mit 2 bis 8 Kohlenstoffatomen und Alkoxycarbonylaminogruppen mit 2 bis 8 Kohlenstoffatomen, wie z.B. Acetylamino, Propionylamino, Methoxycarbonylamino, Aethoxycarbonylamino, Alkanoylgruppen mit 2 bis 8, vorzugsweise 2 bis 4 Kohlenstoffatomen, wie z.B. Acetyl, Propionyl, Butyryl oder Isobutyryl, C₅-C₇-Cycloalkylcarbonyl, wie z.B. Cyclohexylcarbonyl, im Cycloalkylring durch C₁-C₄-Alkyl, wie z.B. Methyl, Aethyl, Propyl, Butyl, oder Halogen, wie z.B. Fluor, Chlor, Brom, Sulfo oder Sulfato, substituiertes C₅-C₇-Cycloalkylcarbonyl, Benzoyl, im Phenylring durch C₁-C₄-Alkyl, wie z.B. Methyl, Aethyl, Propyl, Butyl oder Halogen, wie z.B. Fluor, Chlor, Brom, Sulfo oder Sulfato, substituiertes Benzoyl, gegebenenfalls durch Sulfo substituiertes C₁-C₈-Alkylthio, wie z.B. Methylthio, Aethylthio oder β-Sulfoäthylthio, gegebenenfalls durch C₁-C₄-Alkyl, Halogen, Sulfo oder Sulfato substituiertes Benzthiazol oder Benzoxazol, Benzoylamino, Amino, Mono- oder Dialkylamino mit 1 bis 8 Kohlenstoffatomen im Alkylrest, Phenylamino, Alkoxycarbonyl mit 1 bis 8 Kohlenstoffatomen im Alkoxyrest, C₅-C₇-Cycloalkylaminosulfonyl, Nitro, Cyan, Trifluormethyl, Halogen, wie Fluor, Brom oder insbesondere Chlor, Sulfamoyl, am Stickstoffatom ein- oder zweifach durch C₁-C₄-Alkyl, C₅-C₇-Cycloalkyl, Phenyl oder Naphthyl substituiertes Sulfamoyl, im Phenyl- bzw. Naphthylring durch C₁-C₄-Alkyl, Halogen, wie z.B. Fluor, Brom oder insbesondere Chlor, Sulfo oder Sulfato substituiertes Phenyl- oder Naphthylaminosulfonyl, Carbamoyl, Ureido, Hydroxy, C₁-C₈-Alkylsulfonyl, C₁-C₈-Alkylaminosulfonyl, gegebenenfalls im Phenylring bzw. Naphthylring durch C₁-C₄-Alkyl, Halogen wie z.B. Fluor, Chlor, Brom, Sulfo oder Sulfato substituiertes Phenylsulfonyl oder Naphthylsulfonyl, 1-Azacycloheptan-N-sulfonyl, Carboxy, Sulfomethyl, Sulfo, Sulfato, Thiosulfato, Arylazogruppen wie z.B. die Phenylazo- und Naphthylazogruppe sowie Phenyl, Naphthyl, Phenoxy, Phenoxysulfonyl, Phenylaminosulfonyl, wobei die angegebenen Phenyl- oder Naphthylreste durch die oben angegebenen Substituenten weitersubstituiert sein können. Gegebenenfalls können je zwei benachbarte Substituenten der genannten Ringsysteme weitere ankondensierte Phenylringe oder Cyclohexylringe bilden.

Als C₁-C₈-Alkyl kommt für R₁, R₂, R₃ und R₄ in Formel (1) z.B. in Betracht:

Methyl, Aethyl, Propyl, Isopropyl, Butyl, Isobutyl, sek.-Butyl, tert.-Butyl, Pentyl, Hexyl, Heptyl oder Octyl, sowie die entsprechenden Reste, die z.B. durch Hydroxy, Alkoxy mit 1 bis 4 Kohlenstoffatomen, wie z.B. Methoxy, Aethoxy, Propoxy, Isopropoxy oder Butoxy, Sulfo, Sulfato, Thiosulfato, Cyan oder Halogen, wie z.B. Fluor, Chlor oder Brom, und Phenyl substituiert sind, wobei der Phenylrest durch die oben angegebenen Substituenten weitersubstituiert sein kann.

Bevorzugt sind Azofarbstoffe der Formel (1), worin R₁ Wasserstoff oder C₁-C₄-Alkyl, insbesondere Methyl, ist.

Bevorzugt sind ferner Azofarbstoffe der Formel (1), worin R₂ und R₃ unabhängig voneinander Wasserstoff oder C₁-C₄-Alkyl, insbesondere Methyl, sind.

Bevorzugt sind weiterhin Azofarbstoffe der Formel (1), worin R₄ C₁-C₄-Alkyl, insbesondere Aethyl, oder Benzyl ist.

Bevorzugt sind ferner Azofarbstoffe der Formel (1), worin n die Zahl 1, 2 oder 3, insbesondere 1 oder 2, ist.

Die Azofarbstoffe der Formel (1) enthalten bevorzugt nur eine oder zwei Sulfo-, Sulfato- oder Thiosulfatogruppen, insbesondere zwei Sulfogruppen.

Bevorzugt sind ebenfalls Azofarbstoffe der Formel 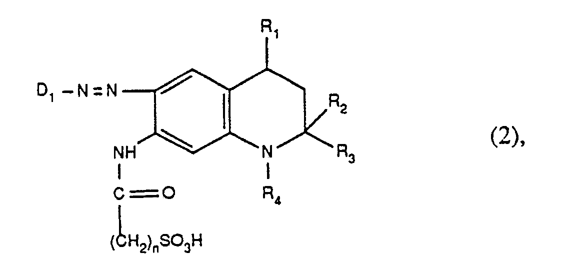 worin D₁ der Rest einer Diazokomponente der Benzol- oder Naphthalinreihe oder der heterocyclischen Reihe ist, insbesondere worin D₁ Thienyl, Thiazolyl, Isothiazolyl, 1,2,4-Thiadiazolyl, 1,3,4-Thiadiazolyl, Benzthiazolyl, Benzisothiazolyl, Benzoxazolyl, Pyrazolyl, Imidazolyl, 1,2,3-Triazolyl, 1,2,4-Triazolyl, Thiophenyl, Benzthiophenyl, Tetrahydrobenzthiophenyl, 7-Oxotetrahydrobenzo[b]thiophenyl, Pyridinyl, Indazolyl, Phenyl oder Naphthyl ist, wobei jeder der genannten Reste substituiert sein kann durch C₁-C₈-Alkyl, C₁-C₈-Alkoxy, C₂-C₈-Alkanoylamino, C₂-C₈-Alkoxycarbonylamino, C₂-C₈-Alkanoyl, C₅-C₇-Cycloalkylcarbonyl, im Cycloalkylring durch C₁-C₄-Alkyl, Halogen, Sulfo oder Sulfato substituiertes C₅-C₇-Cycloalkylcarbonyl, Benzoyl, im Phenylring durch C₁-C₄-Alkyl, Halogen, Sulfo oder Sulfato substituiertes Benzoyl, C₁-C₈-Alkylthio, durch Sulfo substituiertes C₁-C₈-Alkylthio, Benzthiazol, Benzoxazol, durch C₁-C₄-Alkyl, Halogen, Sulfo oder Sulfato substituiertes Benzthiazol oder Benzoxazol, Benzoylamino, Amino, Mono- oder Dialkylamino mit 1 bis 8 Kohlenstoffatomen im Alkylrest, Phenylamino, C₂-C₈-Alkoxycarbonyl, Nitro, Cyan, Trifluormethyl, Halogen, 1-Azacycloheptan-N-sulfonyl, Sulfamoyl, am Stickstoffatom ein- oder zweifach durch C₁-C₄-Alkyl, C₅-C₇-Cycloalkyl, Phenyl oder Naphthyl substituiertes Sulfamoyl, im Phenyl- bzw. Naphthylring durch C₁-C₄-Alkyl, Halogen, Sulfo oder Sulfato substituiertes Phenyl- oder Naphthyl aminosulfonyl, Phenoxy, Phenoxysulfonyl, durch C₁-C₄-Alkyl, Halogen, Sulfo oder Sulfato substituiertes Phenoxy oder Phenoxysulfonyl, Carbamoyl, Ureido, Hydroxy, C₁-C₈-Alkylsulfonyl, Phenylsulfonyl, Naphthylsulfonyl, im Phenylring bzw. Naphthylring durch C₁-C₄-Alkyl, Halogen, Sulfo oder Sulfato substituiertes Phenylsulfonyl oder Naphthylsulfonyl, Carboxy, Sulfomethyl, Sulfo, Sulfato, Thiosulfato und Phenylazo- und Naphthylazogruppen, und gegebenenfalls je 2 benachbarte Substituenten der genannten Ringsysteme weitere ankondensierte Phenylringe oder Cyclohexylringe bilden können und R₁, R₂, R₃, R₄ und n die unter Formel (1) angegebenen Bedeutungen haben, vorzugsweise worin jeder der für D₁ oben genannten Reste substituiert sein kann durch C₁-C₈-Alkyl, C₁-C₈-Alkoxy, C₂-C₈-Alkanoylamino, C₂-C₈-Alkoxycarbonylamino, C₂-C₈-Alkanoyl, C₅-C₇-Cycloalkylcarbonyl, im Cycloalkylring durch C₁-C₄-Alkyl, Halogen, Sulfo oder Sulfato substituiertes C₅-C₇-Cycloalkylcarbonyl, Benzoyl, im Phenylring durch C₁-C₄-Alkyl, Halogen, Sulfo oder Sulfato substituiertes Benzoyl, Benzthiazol, Benzoxazol, durch C₁-C₄-Alkyl, Halogen, Sulfo oder Sulfato substituiertes Benzthiazol oder Benzoxazol, Benzoylamino, Amino, Mono- oder Dialkylamino mit 1 bis 8 Kohlenstoffatomen im Alkylrest, Phenylamino, C₂-C₈-Alkoxycarbonyl, Nitro, Cyan, Trifluormethyl, Halogen, Sulfamoyl, am Stickstoffatom ein- oder zweifach durch C₁-C₄-Alkyl, C₅-C₇-Cycloalkyl oder Phenyl substituiertes Sulfamoyl, Carbamoyl, Ureido, Hydroxy, C₁-C₈-Alkylsulfonyl, Phenylsulfonyl, im Phenylring durch C₁-C₄-Alkyl, Halogen, Sulfo oder Sulfato substituiertes Phenylsulfonyl, Carboxy, Sulfomethyl, Sulfo, Sulfato, Thiosulfato und Phenylazo- und Naphthylazogruppen, und gegebenenfalls je 2 benachbarte Substituenten der genannten Ringsysteme weitere ankondensierte Phenylringe oder Cyclohexylringe bilden können und R₁, R₂, R₃, R₄ und n die unter Formel (1) angegebenen Bedeutungen haben.

Besonders bevorzugt sind Azofarbstoffe der Formel 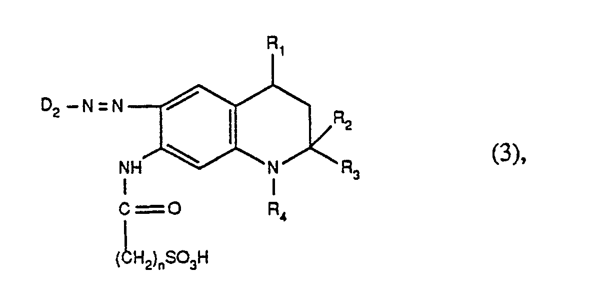 worin D₂ Thiophenyl, Benzthiophenyl, Benzisothiazolyl, 1,3,4-Thiadiazolyl, 7-Oxotetra hydrobenzo[b]thiophenyl oder Phenyl ist, wobei jeder der genannten Reste substituiert sein kann durch C₁-C₈-Alkyl, C₁-C₈-Alkoxy, C₂-C₈-Alkanoyl, Carbamoyl, C₂-C₈-Alkoxycarbonyl, C₅-C₇-Cycloalkylcarbonyl, Benzoyl, im Phenylring durch C₁-C₄-Alkyl, Halogen, Sulfo oder Sulfato substituiertes Benzoyl, C₁-C₈-Alkylthio, durch Sulfo substituiertes C₁-C₈-Alkylthio, Benzthiazol, Benzoxazol, durch C₁-C₄-Alkyl, Halogen, Sulfo oder Sulfato substituiertes Benzthiazol oder Benzoxazol, Halogen, Sulfo, Trifluormethyl, Phenylsulfonyl, Naphthylsulfonyl, im Phenylring bzw. Naphthylring durch C₁-C₄-Alkyl, Halogen, Sulfo oder Sulfato substituiertes Phenylsulfonyl oder Naphthylsulfonyl, C₁-C₈-Alkylsulfonyl, 1-Azacycloheptan-N-sulfonyl, Sulfamoyl, am Stickstoffatom ein- oder zweifach durch C₁-C₄-Alkyl, C₅-C₇-Cycloalkyl, Phenyl oder Naphthyl substituiertes Sulfamoyl, im Phenyl- bzw. Naphthylring durch C₁-C₄-Alkyl, Halogen, Sulfo oder Sulfato substituiertes Phenyl- oder Naphthylaminosulfonyl, Phenoxy, Phenoxysulfonyl, durch C₁-C₄-Alkyl, Halogen, Sulfo oder Sulfato substituiertes Phenoxy oder Phenoxysulfonyl und R₁, R₂, R₃, R₄ und n die unter Formel (1) angegebenen Bedeutungen haben, vorzugsweise worin jeder der oben für D₂ genannten Reste substituiert sein kann durch C₁-C₈-Alkyl, C₂-C₈-Alkanoyl, C₂-C₈-Alkoxycarbonyl, C₅-C₇-Cycloalkylcarbonyl, Benzoyl, im Phenylring durch C₁-C₄-Alkyl, Halogen, Sulfo oder Sulfato substituiertes Benzoyl, Benzthiazol, Benzoxazol, durch C₁-C₄-Alkyl, Halogen, Sulfo oder Sulfato substituiertes Benzthiazol oder Benzoxazol, Halogen, Phenylsulfonyl, im Phenylring durch C₁-C₄-Alkyl, Halogen, Sulfo oder Sulfato substituiertes Phenylsulfonyl, C₁-C₈-Alkylsulfonyl, am Stickstoffatom ein- oder zweifach durch C₁-C₄-Alkyl, C₅-C₇-Cycloalkyl oder Phenyl substituiertes Sulfamoyl und R₁, R₂, R₃, R₄ und n die unter Formel (1) angegebenen Bedeutungen haben.

Besonders bevorzugt sind Azofarbstoffe der Formel (1), worin R₁ Wasserstoff oder C₁-C₄-Alkyl, insbesondere Methyl, R₂ und R₃ unabhängig voneinander Wasserstoff oder C₁-C₄-Alkyl, insbesondere Methyl, R₄ C₁-C₄-Alkyl, insbesondere Aethyl, n die Zahl 1 oder 2 ist und D die unter Formel (1) angegebenen Bedeutungen hat, insbesondere worin D die für D₁ unter Formel (2) angegebenen Bedeutungen hat, vorzugsweise hat D die für D₂ unter Formel (3) angegebenen Bedeutungen.

Interessant sind ferner Azofarbstoffe der Formel (2), worin R₁ Wasserstoff oder Methyl, R₂ und R₃ unabhängig voneinander Wasserstoff oder Methyl, R₄ Aethyl, n die Zahl 1 oder 2 ist, und D₁ die unter Formel (2) angegebenen Bedeutungen, insbesondere die für D₂ unter Formel (3) angegebenen Bedeutungen, hat.

Ganz besonders bevorzugt sind Azofarbstoffe der Formel 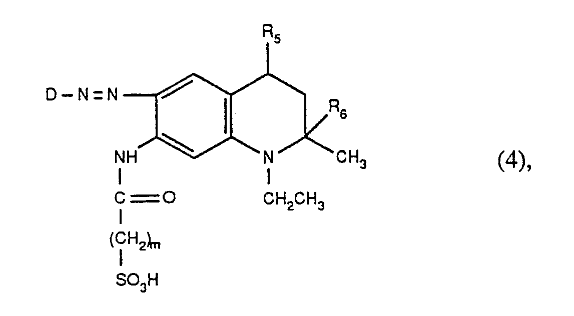 worin R₅ und R₆ unabhängig voneinander Wasserstoff oder Methyl, m die Zahl 1 oder 2 ist und D die unter Formel (1) angegebenen Bedeutungen hat, insbesondere worin D die für D₁ unter Formel (2) angegebenen Bedeutungen hat.

Vorzugsweise hat D in Formel (4) die für D₂ unter Formel (3) angegebenen Bedeutungen.

Besonders interessant sind Azofarbstoffe der Formel (4), worin R₅ und R₆ beide Wasserstoff oder R₅ und R₆ beide Methyl bedeuten.

Ganz besonders wichtig sind Azofarbstoffe der Formel (4), worin R₅ und R₆ beide Wasserstoff oder R₅ und R₆ beide Methyl bedeuten und worin m die Zahl 1 oder 2 ist und D die für D₁ unter Formel (2) angegebenen Bedeutungen, insbesondere die für D₂ unter Formel (3) angegebenen Bedeutungen, hat.

Gegenstand der Erfindung ist ferner ein Verfahren zur Herstellung der Azofarbstoffe der Formel (1), welches dadurch gekennzeichnet ist, dass man ein Amin der Formel
D-NH₂ (5)
diazotiert und auf eine Kupplungskomponente der Formel 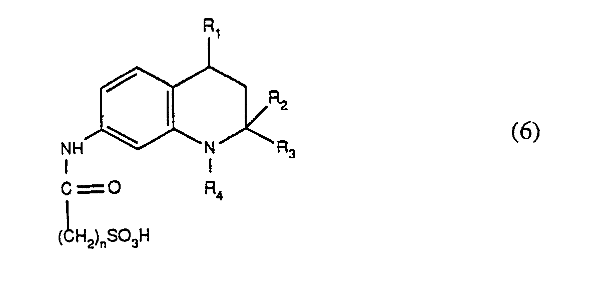 kuppelt, wobei D, R₁, R₂, R₃, R₄ und n die unter Formel (1) angegebenen Bedeutungen haben.

Gegebenenfalls kann nach der Kupplung noch eine wasserlöslichmachende Gruppe in den Azofarbstoff der Formel (1) eingeführt werden; ferner kann im Anschluss an die Kupplung noch eine Alkylierung oder Acylierung vorgenommen werden.

Die Diazotierung der Diazokomponente der Formel (5) erfolgt in der Regel durch Einwirken salpetriger Säure in wässrig-mineralsaurer Lösung bei tiefer Temperatur, die Kupplung auf die Kupplungskomponente der Formel (6) bei sauren bis neutralen pH-Werten.

Gegebenenfalls kann eine freie Aminogruppe im Rest D nach der Kupplung mit einem Acylierungs- oder Alkylierungsmittel in eine Acylamino- oder Alkylaminogruppe umgewandelt werden, und ebenso kann eine Hydroxygruppe durch Acylierung oder Alkylierung in eine Acyloxy oder Alkoxygruppe überführt werden.

Ferner kann eine freie Hydroxygruppe in eine wasserlöslichmachende Gruppe, wie z.B. durch Sulfatierung in eine Sulfatogruppe, überführt werden.

Vorzugsweise werden für das erfindungsgemässe Verfahren Amine der Formel (5) verwendet, worin D die für D₁ unter Formel (2) angegebenen Bedeutungen, insbesondere die für D₂ unter Formel (3) angegebenen Bedeutungen, hat.

Bevorzugt für das erfindungsgemässe Verfahren sind Kupplungskomponenten der Formel (6), worin R₁ Wasserstoff oder C₁-C₄-Alkyl, insbesondere Methyl, ist.

Bevorzugt für das erfindungsgemässe Verfahren sind ferner Kupplungskomponenten der Formel (6), worin R₂ und R₃ unabhängig voneinander Wasserstoff oder C₁-C₄-Alkyl, insbesondere Methyl, sind.

Bevorzugt für das erfindungsgemässe Verfahren sind weiterhin Kupplungskomponenten der Formel (6), worin R₄ C₁-C₄-Alkyl, insbesondere Aethyl, oder Benzyl ist.

Bevorzugt für das erfindungsgemässe Verfahren sind ferner Kupplungskomponenten der Formel (6), worin n die Zahl 1, 2 oder 3, insbesondere 1 oder 2, ist.

Eine bevorzugte Ausführungsform des erfindungsgemässen Verfahrens zur Herstellung der Azofarbstoffe der Formel (1) ist dadurch gekennzeichnet, dass man ein Amin der Formel (5), worin D die unter Formel (1) angegebenen Bedeutungen, insbesondere die für D₁ unter Formel (2) angegebenen Bedeutungen, hat, diazotiert, und auf eine Kupplungskomponente der Formel (6), worin R₁ Wasserstoff oder C₁-C₄-Alkyl, insbesondere Methyl, R₂ und R₃ unabhängig voneinander Wasserstoff oder C₁-C₄-Alkyl, insbesondere Methyl, R₄ C₁-C₄-Alkyl, insbesondere Aethyl, und n die Zahl 1 oder 2 ist, kuppelt. Vorzugsweise verwendet man ein Amin der Formel (5), worin D die für D₂ unter Formel (3) angegebenen Bedeutungen hat.

Die ganz besonders bevorzugten Azofarbstoffe der Formel (4) werden hergestellt, indem man ein Amin der Formel (5), worin D die unter Formel (1) angegebenen Bedeutungen, insbesondere die für D₁ unter Formel (2) angegebenen Bedeutungen, hat, diazotiert und auf eine Kupplungskomponente der Formel 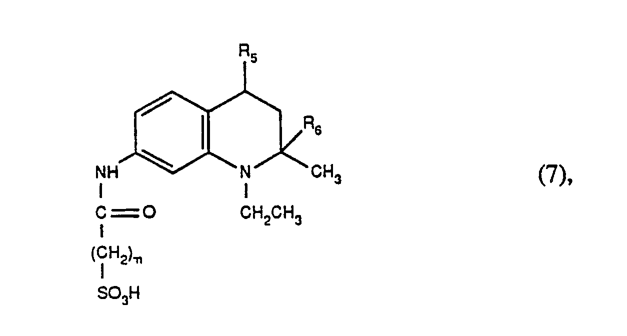 worin R₅ und R₆ unabhängig voneinander Wasserstoff oder Methyl und m die Zahl 1 oder 2 ist, kuppelt. Vorzugsweise verwendet man für das erfindungsgemässe Verfahren zur Herstellung der Azofarbstoffe der Formel (4) ein Amin der Formel (5), worin D die für D₂ unter Formel (3) angegebenen Bedeutungen hat.

Eine bevorzugte Ausführungsform des erfindungsgemässen Verfahrens zur Herstellung der Azofarbstoffe der Formel (4) ist dadurch gekennzeichnet, dass man eine Kupplungskomponente der Formel (7) verwendet, worin R₅ und R₆ beide Wasserstoff oder R₅ und R₆ beide Methyl bedeuten.

Eine besonders bevorzugte Ausführungsform des erfindungsgemässen Verfahrens zur Herstellung der Azofarbstoffe der Formel (4) ist dadurch gekennzeichnet, dass man eine Kupplungskomponente der Formel (7), worin R₅ und R₆ beide Wasserstoff oder R₅ und R₆ beide Methyl bedeuten und worin m die Zahl 1 oder 2 ist, verwendet.

Aus der grossen Zahl möglicher Amine der Formel (5) kommen z.B. in Betracht:
2-Amino-3-methoxycarbonylthiophen,
2-Amino-3-äthoxycarbonylthiophen,
2-Amino-3-methoxycarbonyl-5-isobutyrylthiophen,
2-Amino-3,5-dimethoxycarbonylthiophen,
2-Amino-3,5-dimethoxycarbonyl-4-methylthiophen,
2-Amino-3,5-diäthoxycarbonyl-4-methylthiophen,
2-Amino-3-methoxycarbonyl-5-(p-methylbenzoyl-)thiophen,
2-Amino-3-methoxycarbonyl-5-cyclohexylcarbonylthiophen,
2-Amino-3-äthoxycarbonyl-5-(benzthiazol-2′-yl-)thiophen,
2-Amino-3-methoxycarbonyl-5-(benzthiazol-2′-yl-)thiophen,
2-Amino-3-äthoxycarbonyl-5-(benzoxazol-2′-yl-)thiophen,
2-Amino-3-methoxycarbonyl-5-(benzoxazol-2′-yl-)thiophen,
2-Amino-3-methoxycarbonyl-4-methyl-5-(benzthiazol-2′-yl-)thiophen,
2-Amino-3-äthoxycarbonyl-4-methyl-5-(benzthiazol-2′-yl-)thiophen,
2-Amino-3-methoxycarbonyl-4-methyl-5-(benzoxazol-2′-yl-)thiophen,
2-Amino-3-äthoxycarbonyl-4-methyl-5-(benzoxazol-2′-yl-)thiophen,
2-Chlor-4-methylsulfonylanilin, 4-Methylsulfonylanilin,
2-Chlor-4-äthylsulfonylanilin,
4-Aethylsulfonylanilin,
2-Phenylsulfonylanilin,
4-Phenylsulfonylanilin,
2-(p-Chlorphenyl-)sulfonyl-5-äthylsulfonylanilin,
2-(1-Azacycloheptan-N-sulfonyl)anilin,
7-Oxotetrahydrobenzo[b]-2-amino-3-methoxycarbonyl-6-methylthiophen,
7-Oxotetrahydrobenzo[b]-2-amino-3-methoxycarbonylthiophen,
7-Oxotetrahydrobenzo[b]-2-amino-3-äthoxycarbonyl-6-methylthiophen,
7-Oxotetrahydrobenzo[b]-2-amino-3-äthoxycarbonylthiophen,
2-Amino-3-methoxycarbonyl-5-(5′- oder 6′-sulfobenzthiazol-2′-yl)-thiophen,
2-Amino-3-äthoxycarbonyl-5-(5′- oder 6′-sulfobenzthiazol-2′-yl)-thiophen,
2-Amino-3-carbamoyl-5-(5′- oder 6′-sulfobenzthiazol-2′-yl)-thiophen,
2-Amino-3-carbamoyl-5-(benzthiazol-2′-yl)-thiophen,
2-Amino-3-methoxycarbonyl-5-(5′- oder 6′-sulfobenzoxazol-2′-yl)thiophen,
2-Amino-3-äthoxycarbonyl-5-(5′- oder 6′-sulfobenzoxazol-2′-yl)thiophen,
2-Amino-3-carbamoyl-5-(5′- oder 6′-sulfobenzoxazol-2′-yl)thiophen,
2-Amino-3-carbamoyl-5-(benzoxazol-2′-yl)thiophen,
2-Methoxycarbonyl-3-aminobenzthiophen,
2-Methoxycarbonyl-3-amino-4-chlorobenzthiophen,
2-Methoxycarbonyl-3-aminobenzthiophen-4-, -5-, -6- oder -7-sulfonsäure,
2-Amino-5-äthylthio-1,3,4-thiadiazol,
2-Amino-5-(β-sulfoäthylthio)1,3,4-thiadiazol,
3-Aminobenzisothiazol,
3-Aminobenzisothiazol-7-sulfonsäure,
3-Amino-4-, -5-, -6- oder -7-chlorobenzisothiazol,
2-(2′-Chlorophenylsulfonyl)anilin,
3-Phenylaminosulfonyl-4-methylanilin,
4-Sulfoamoylanilin,
2-(2′-Chlorophenoxy)-5-chloroanilin,
2-Trifluoromethyl-4-chloroanilin,
2-Trifluoromethylanilin,
2,5-Dimethoxy-4-phenylaminosulfonylanilin,
2-Methylsulfonylanilin,
2-(2′-Amino-4′-sulfamoylphenylsulfonyl)-naphthalin-5-, -6-, -7- oder -8-sulfonsäure,
2-Amino-4′-methyl-4-(1˝-sulfonaphth-2˝-ylaminosulfonyl)-1,1′-diphenylsulfon,
2-Amino-4′-methyl-4-(3˝-sulfophenylaminosulfonyl)-1,1′-diphenylsulfon,
2-Amino-4-sulfamoyl-3′-sulfo-4′-methyl-1,1′-diphenylsulfon,
2-Amino-3′-sulfo-4′-methyl-4-dimethylaminosulfonyl-1,1′-diphenylsulfon,
2-Amino-4-sulfo-4′-methyl-1,1′-diphenylsulfon,
2-Amino-4′-methyl-4-(4˝-sulfophenoxysulfonyl)-1,1′-diphenylsulfon.

Die Amine der Formel (5) sind an sich bekannt und können in Analogie zu bekannten Verbindungen hergestellt werden.

Die Amine der Formel (5) können in Aminogruppen überführbare Reste, wie z.B. die Acetylamino- und die Nitrogruppe, enthalten. Beispielsweise kann eine Acetylaminogruppe durch Verseifen und eine Nitrogruppe durch Reduktion in eine Aminogruppe überführt werden.

Ferner kann eine wasserlöslichmachende Gruppe im Anschluss an Diazotierung und Kupplung in die Azofarbstoffe eingeführt werden, z.B. durch Sulfatierung einer Hydroxygruppe im Amin der Formel (5) und/oder in der Kupplungskomponente der Formel (6).

Die Verbindungen der Formel (6), worin R₁, R₂, R₃, R₄ und n die unter Formel (1) angegebenen Bedeutungen haben, sind neu und stellen einen weiteren Gegenstand der Erfindung dar.

Bevorzugt sind Verbindungen der Formel (6), worin R₁ Wasserstoff oder C₁-C₄-Alkyl, insbesondere Methyl, ist.

Bevorzugt sind ferner Verbindungen der Formel (6), worin R₂ und R₃ unabhängig voneinander Wasserstoff oder C₁-C₄-Alkyl, insbesondere Methyl, sind. Bevorzugt sind weiterhin Verbindungen der Formel (6), worin R₄ C₁-C₄-Alkyl, insbesondere Aethyl, oder Benzyl ist.

Ebenfalls bevorzugt sind Verbindungen der Formel (6), worin n die Zahl 1, 2 oder 3, insbesondere die Zahl 1 oder 2, ist.

Besonders bevorzugt sind Verbindungen der Formel (6), worin R₁ Wasserstoff oder C₁-C₄-Alkyl, insbesondere Methyl, R₂ und R₃ unabhängig voneinander Wasserstoff oder C₁-C₄-Alkyl, insbesondere Methyl, R₄ C₁-C₄-Alkyl, insbesondere Aethyl, und n die Zahl 1 oder 2 ist.

Ganz besonders bevorzugt sind Verbindungen der Formel (7), worin R₅, R₆ und m die unter Formel (7) angegebenen Bedeutungen haben.

Ganz besonders wichtig sind Verbindungen der Formel (7), worin R₅ und R₆ beide Wasserstoff oder R₅ und R₆ beide Methyl bedeuten und worin m die Zahl 1 oder 2 ist.

Ein weiterer Gegenstand der Erfindung ist ein Verfahren zur Herstellung der Verbindungen der Formel (6), welches dadurch gekennzeichnet ist, dass man eine Verbindung der Formel 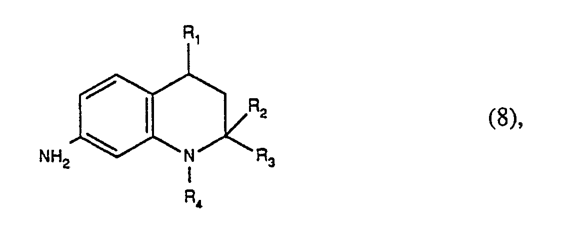 worin R₁, R₂, R₃ und R₄ die unter Formel (1) angegebenen Bedeutungen haben, mit einer Verbindung der Formel

X-(CH₂)ₙ- 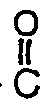 -Y (9) oder

(X-(CH₂)ₙ- 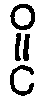 )₂O (10),
worin X und Y je eine anionische Abgangsgruppe bedeuten und n die unter Formel (1) angegebenen Bedeutungen hat, zu einer Verbindung der Formel 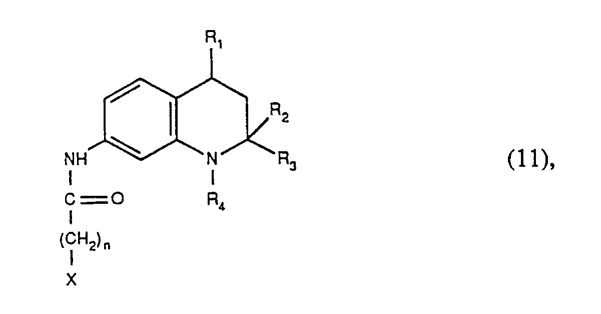 umsetzt, worin R₁, R₂, R₃, R₄ und n die unter Formel (1) angegebenen Bedeutungen und X die unter Formel (9) angegebene Bedeutung hat, und die Verbindung der Formel (11) durch Austausch des Restes X durch eine Sulfogruppe zu einer Verbindung der Formel (6) umsetzt.

Als X und Y in Formel (9) sowie als X in Formel (10) kommen unabhängig voneinander z.B. Halogen, wie z.B. Fluor, Chlor oder Brom, in Betracht. Insbesondere sind X und Y jeweils Chlor. Als Beispiele für Verbindungen der Formel (9) seien Chloressigsäurechlorid, 3-Chlorpropionsäurechlorid, 4-Chlorbuttersäurechlorid und 5-Valeriansäurechlorid genannt. Als Beispiele der Verbindung der Formel (10) seien die entsprechenden Anhydride, wie z.B. Chloressigsäureanhydrid, genannt.

Die Umsetzung der Verbindung der Formel (8) mit der Verbindung der Formel (9) oder (10) erfolgt in Wasser oder organischen Lösungsmitteln, wie z.B. Tetrahydrofuran, Dioxan oder Benzol, bei Temperaturen von -10 bis 80°C, insbesondere bei Temperaturen von 0 bis 40°C.

Die Einführung der Sulfogruppe zur Verbindung der Formel (11) erfolgt durch Austausch des Restes X durch eine Sulfogruppe, wie z.B. mit Na₂SO₃, in einem Lösungsmittel, wie z.B. Wasser oder einer Wasser/Aethanolmischung, bei Temperaturen von 60 bis 130°C, insbesondere bei Temperaturen von 85 bis 100°C, bei saurem, neutralem bis alkalischem pH-Wert, insbesondere einem pH-Wert von 5 bis 9, sowie gegebenenfalls unter Druck, wie z.B. einem Druck von 1 bis 5 bar.

Eine bevorzugte Ausführungsform des erfindungsgemässen Verfahrens zur Herstellung der Verbindungen der Formel (6) ist dadurch gekennzeichnet, dass man eine Verbindung der Formel (8), worin R₁ Wasserstoff oder C₁-C₄-Alkyl, insbesondere Methyl, R₂ und R₃ unabhängig voneinander Wasserstoff oder C₁-C₄-Alkyl, insbesondere Methyl, und R₄ Benzyl oder C₁-C₄-Alkyl, insbesondere Aethyl, ist, mit einer Verbindung der Formel (9), worin X und Y jeweils Chlor sind und n die Zahl 1, 2 oder 3, insbesondere die Zahl 1 oder 2, ist, zu einer Verbindung der Formel (11), worin R₁, R₂, R₃ und R₄ die für Formel (8) und X und n die für Formel (9) der bevorzugten Ausführungsform angegebenen Bedeutungen haben, umsetzt, und die Verbindung der Formel (11) durch Sulfonierung, insbesondere mit Na₂SO₃, zu einer Verbindung der Formel (6) umsetzt.

Eine ganz besonders bevorzugte Ausführungsform des erfindungsgemässen Verfahrens zur Herstellung der Verbindungen der Formel (6) ist dadurch gekennzeichnet, dass man Verbindungen der Formel (8) verwendet, worin R₁ und R₂ unabhängig voneinander Wasserstoff oder Methyl, R₃ Methyl und R₄ Aethyl ist, vorzugsweise Verbindungen der Formel (8), worin R₁ und R₂ beide Wasserstoff oder R₁ und R₂ beide Methyl sind, R₃ Methyl und R₄ Aethyl ist, verwendet.

Die Farbstoffe der Formel (1) liegen entweder in der Form ihrer freien Säure oder vorzugsweise als deren Salze vor.

Als Salze kommen beispielsweise die Alkali- oder Ammoniumsalze oder die Salze eines organischen Amins in Betracht.

Als Beispiel seien die Natrium-, Lithium-, Kalium- oder Ammoniumsalze oder das Salz des Mono-, Di- oder Triäthanolamins genannt.

Die erfindungsgemässen Azofarbstoffe der Formel (1) eignen sich nach an sich bekannten Methoden zum Färben und Bedrucken, insbesondere von stickstoffhaltigen oder hydroxygruppenhaltigen Fasermaterialien, wie z.B. textilen Fasermaterialien aus Cellulose, Seide und insbesondere Wolle und synthetischen Polyamiden. Man erhält egale Färbungen in roten und blauen Tönen mit guten Allgemeinechtheiten, insbesondere guter Reib-, Nass-, Nassreib- und Lichtechtheit. Ferner sind die erfindungsgemässen Farbstoffe sehr gut mit anderen Farbstoffen kombinierbar. Das oben genannte Textilmaterial kann in den verschiedensten Verarbeitungsformen vorliegen, wie z.B. als Faser, Garn, Gewebe oder Gewirke.

In den folgenden Beispielen stehen Teile für Gewichtsteile. Die Temperaturen sind in Celsiusgraden angegeben. Die Beziehung zwischen Gewichtsteilen und Volumenteilen ist dieselbe wie diejenige zwischen Gramm und Kubikzentimeter.

### Beispiel 1:

Bei Raumtemperatur werden 91,4 Teile 7-Amino-1-äthyl- 1,2,3,4-tetrahydro-2,2,4-trimethylchinolin in 285 Teilen Tetrahydrofuran gelöst und unter Rühren 106,6 Teile wasserfreies Natriumacetat innerhalb von 2 Minuten eingetragen.

Innerhalb 30 Minuten werden danach 64,2 Teile Chloracetylchlorid unter Eiskühlung bei einer Innentemperatur von 20° zugetropft. Es wird 1 Stunde bei 20° gerührt und danach 250 Teile Wasser zugetropft. Man rührt 10 Minuten bei 20° nach, trennt danach die wässrige Phase ab und schüttelt die organische Phase einmal mit 100 Teilen 15%-iger wässriger Natriumchlorid-Lösung aus. Die organische Phase wird anschliessend über Natriumsulfat getrocknet und das Rohprodukt durch Einengen der Lösung am Rotationsverdampfer gewonnen. Man erhält 133,1 Teile eines grauen Pulvers der Formel

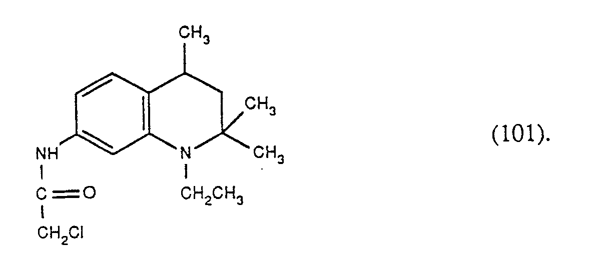

### Beispiel 2:

Verfährt man wie in Beispiel 1 angegeben, verwendet jedoch statt 91,4 Teile 7-Amino-1-äthyl-1,2,3,4-tetrahydro-2,2,4-trimethylchinolin eine äquimolare Menge 7-Amino-1-äthyl-1,2,3,4-tetrahydro-2-methylchinolin, so erhält man eine Verbindung der Formel

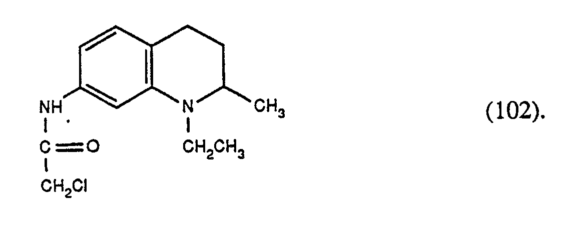

### Beispiel 3:

Bei Raumtemperatur werden 58,9 Teile 7-Amino-1-äthyl-1,2,3,4-tetrahydro-2,2,4-trimethylchinolin in 142 Teilen Tetrahydrofuran gelöst. Danach gibt man 53 Teile wasserfreies Natriumacetat zu, kühlt auf 0° ab und tropft 36 Teile Chlorpropionsäurechlorid innerhalb 30 Minuten bei einer Innentemperatur von 0 bis 3° zu. Es wird 1 Stunde bei 0 bis 5° nachgerührt und anschliessend 125 Teile Wasser zugetropft. Es wird 10 Minuten bei 5° nachgerührt und danach die wässrige Phase im Scheidetrichter abgetrennt. Die organische Phase wird einmal mit 50 Teilen Wasser gewaschen und anschliessend über Natriumsulfat getrocknet und das Rohprodukt durch Einengen der Lösung am Rotationsverdampfer gewonnen. Man erhält 74,4 Teile eines grauen Pulvers der Formel

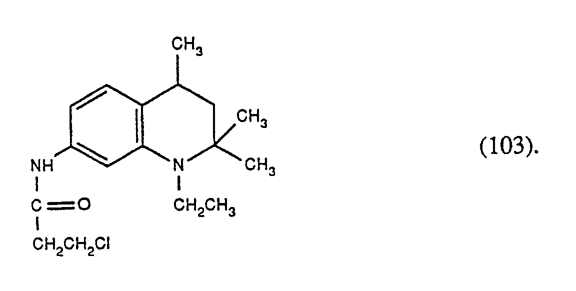

### Beispiel 4:

Verfährt man wie in Beispiel 3 angegeben, verwendet jedoch statt 58,9 Teile 7-Amino-1-äthyl-1,2,3,4-tetrahydro-2,2,4-trimethylchinolin eine äquimolare Menge 7-Amino-1-äthyl-1,2,3,4-tetrahydro-2-methylchinolin, so erhält man eine Verbindung der Formel

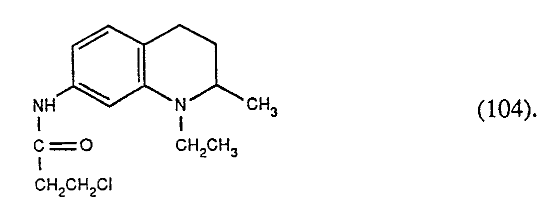

### Beispiel 5:

Bei Raumtemperatur werden 133,1 Teile der nach Beispiel 1 hergestellten Verbindung der Formel (101) unter Rühren in 600 Teilen Wasser eingetragen. Anschliessend wird der pH-Wert durch Zugabe von 30%-iger wässriger Natronlauge auf 7 gestellt und 65 Teile Natriumsulfit eingetragen. Danach wird die Temperatur auf ca. 95° erhöht und 4 Stunden bei 93 bis 95° gerührt. Anschliessend lässt man auf 70° abkühlen und streut innerhalb 3 Minuten 105 Teile Natriumchlorid ein. Danach rührt man ohne zu heizen 1 Stunde nach, kühlt auf 15° ab und filtriert. Das abfiltrierte Rohprodukt wird 12 Stunden im Vakuum bei 60° getrocknet und anschliessend in 275 Teile absoluten Essigester eingerührt. Nach 15 Minuten Rühren wird das Produkt über eine Nutsche abgesaugt, mit 50 Teilen absolutem Essigester nachgewaschen und an der Luft getrocknet.

Man erhält 186 Teile eines grauen Pulvers der Formel 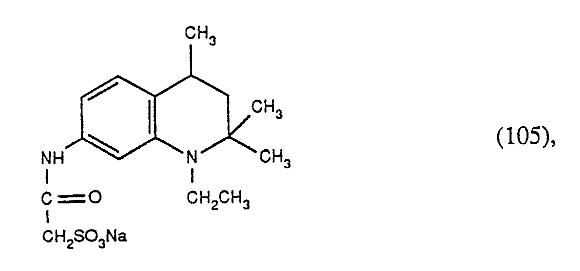 mit einer Ausbeute von ca. 75 % der Theorie.

### Beispiel 6:

Verfährt man wie in Beispiel 5 angegeben, verwendet jedoch statt 133,1 Teile der Verbindung der Formel (101) eine äquimolare Menge der Verbindung der Formel (102), so erhält man eine Verbindung der Formel

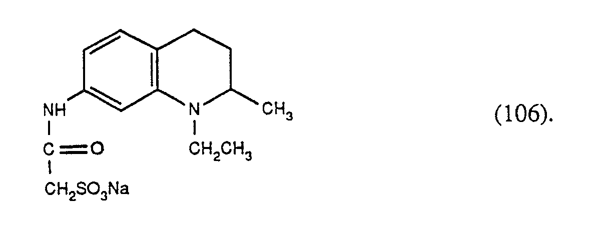

### Beispiel 7:

Bei Raumtemperatur werden 74,4 Teile der nach Beispiel 3 hergestellten Verbindung der Formel (103) in 300 Teile Wasser eingetragen und der pH-Wert durch Zugabe von 30%-iger wässriger Natronlauge auf 7,5 gestellt. Danach gibt man 38 Teile Natriumsulfit zu und erhöht die Temperatur auf 97°. Man erhitzt 12 Stunden bei 97° und hält den pH-Wert durch Zugabe von 30%-iger wässriger Natronlauge auf einen Wert zwischen 6,8 und 7,0. Nach 12 Stunden gibt man 60 Teile Aethanol zu und rührt nochmals 12 Stunden bei 86 bis 88°. Danach kühlt man auf 70° ab, gibt 200 Teile Wasser zu und trägt innerhalb 2 Minuten 80 Teile Natriumchlorid ein. Man rührt 1 Stunde ohne zu heizen nach, kühlt auf 15° ab und filtriert das Rohprodukt ab. Nach Trocknung im Vakuum erhält man 92 Teile eines Rohproduktes der Formel

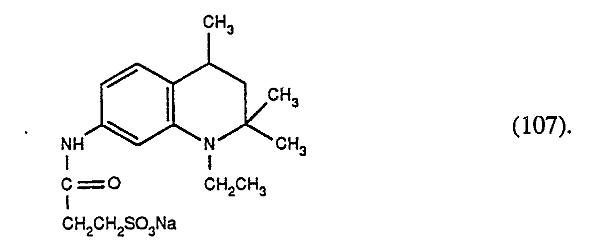

Das Rohprodukt kann durch Umkristallisieren aus Aceton gereinigt werden.

### Beispiel 8:

Verfährt man wie in Beispiel 7 angegeben, verwendet jedoch statt 74,4 Teile der Verbindung der Formel (103) eine äquimolare Menge der Verbindung der Formel (104), so erhält man eine Verbindung der Formel

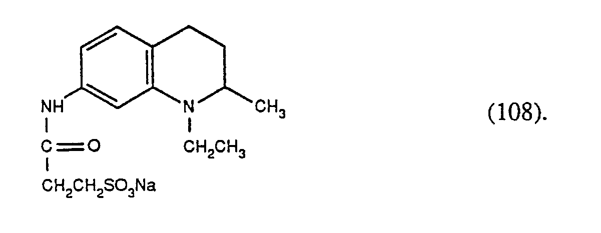

### Beispiel 9:

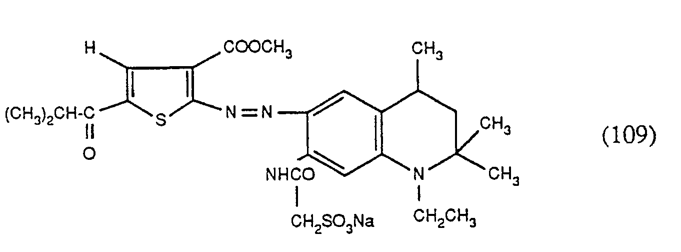

Bei 10° werden 25 Teile Eisessig und 12 Teile Salzsäure (32 %) unter Rühren vorgelegt. Danach werden 2,3 Teile 2-Amino-3-carbomethoxy-5-isobutyryl-thiophen eingetragen und die Temperatur auf 0° gesenkt. Bei 0 bis 2° wird danach eine Lösung von 0,74 Teilen NaNO₂ und 4 Teilen Wasser innerhalb 3 Minuten zugetropft und 15 Minuten nachgerührt. Nach Zugabe von 1 Teil Sulfaminsäure wird kurz nachgerührt und 3,65 Teile der Kupplungskomponente der Formel (105) innerhalb 1 Minute eingetragen. Man rührt anschliessend 5 Min. bei 0 bis 2° nach und gibt dann innerhalb der nächsten 20 Minuten 24 Teile Natriumacetat x 3H₂O in 4 Portionen zu. Danach wird noch 30 Minuten bei 0° nachgerührt und anschliessend eine Lösung von 6 Teilen Natriumacetat, 3 Teilen Soda und 30 Teilen Wasser innerhalb 20 Minuten bei einer Innentemperatur von 0 bis 3° zugetropft. Nach Filtration bei Raumtemperatur wird der Farbstoff bei 50° im Vakuum getrocknet. Man erhält 6,7 Teile eines schwarzen Pulvers der Verbindung der Formel (109), das natürliches und synthetisches Polyamidfasermaterial in blauen Farbtönen färbt.

### Beispiele 10 bis 30:

Verfährt man wie in Beispiel 9 angegeben, verwendet jedoch statt 2,3 Teile 2-Amino-3-carbomethoxy-5-isobutyrylthiophen eine äquimolare Menge der in Tabelle 1 in Spalte 2 angegebenen Amine und gegebenenfalls statt 3,65 Teilen der Kupplungskomponente der Formel (105) eine äquimolare Menge einer Kupplungskomponente der Formel (107), so erhält man die in Spalte 3 in Form der freien Säuren angegebenen Azofarbstoffe, die natürliches und synthetisches Polyamidfasermaterial in den in Spalte 4 angegebenen Farbtönen färben.

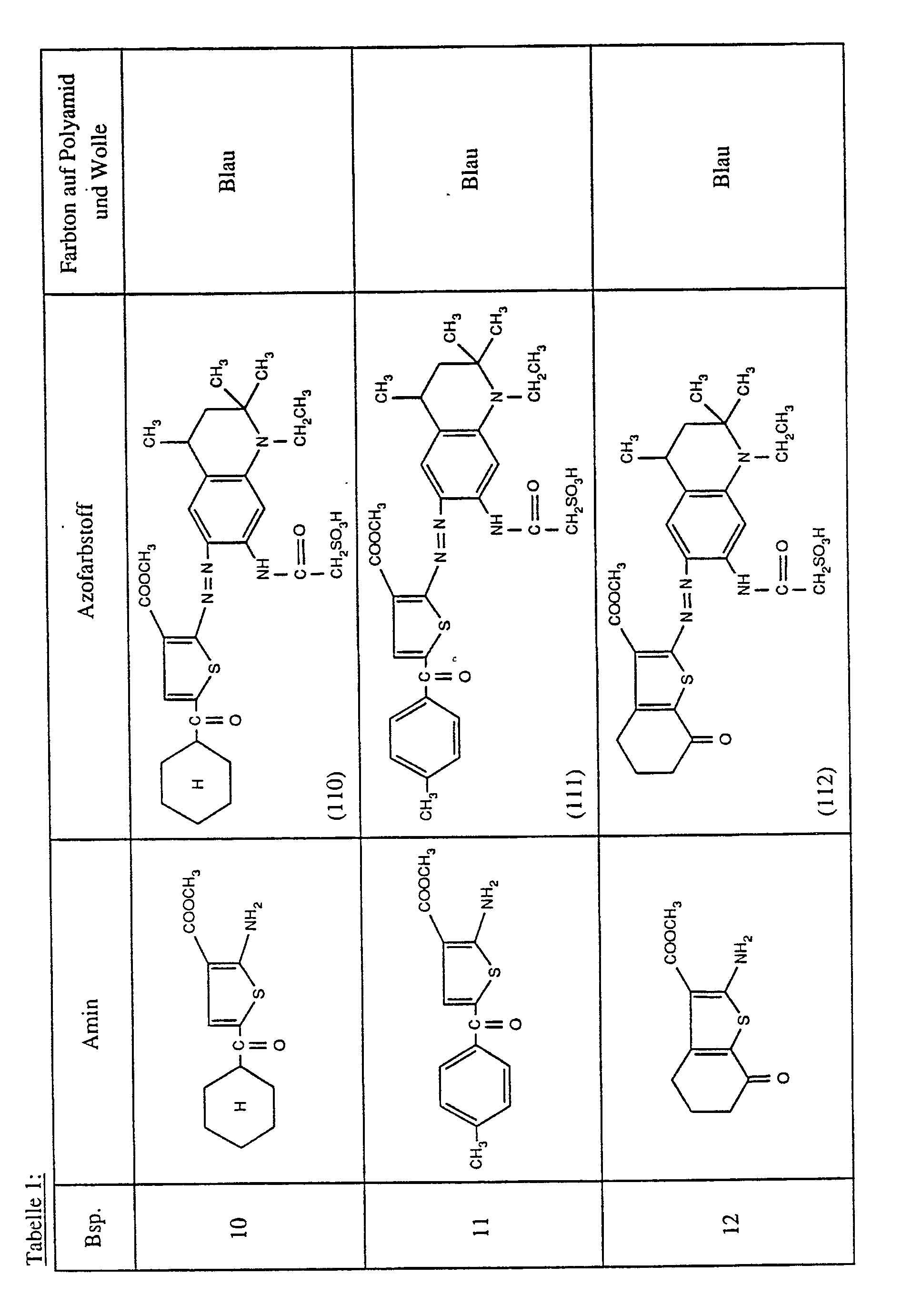

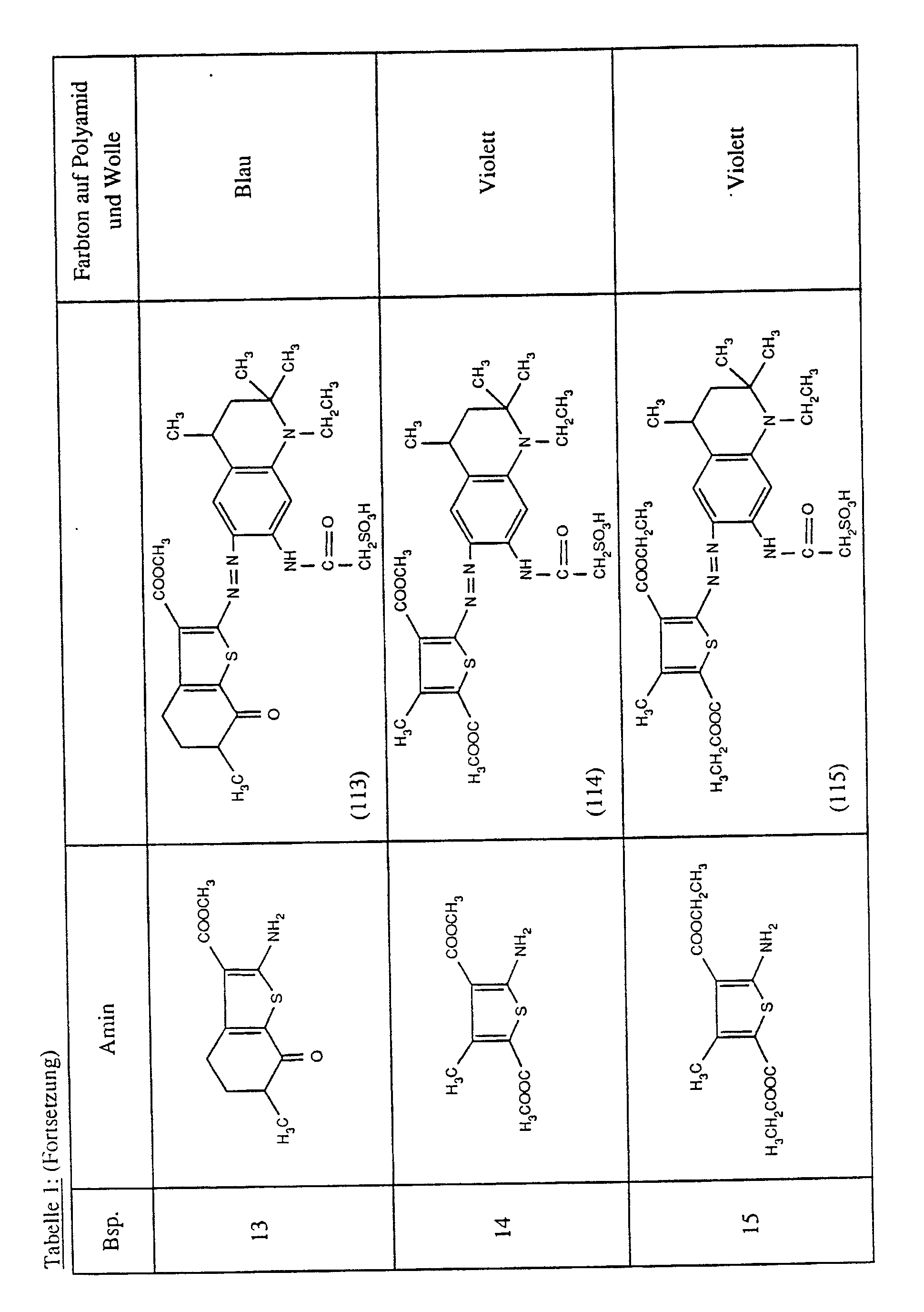

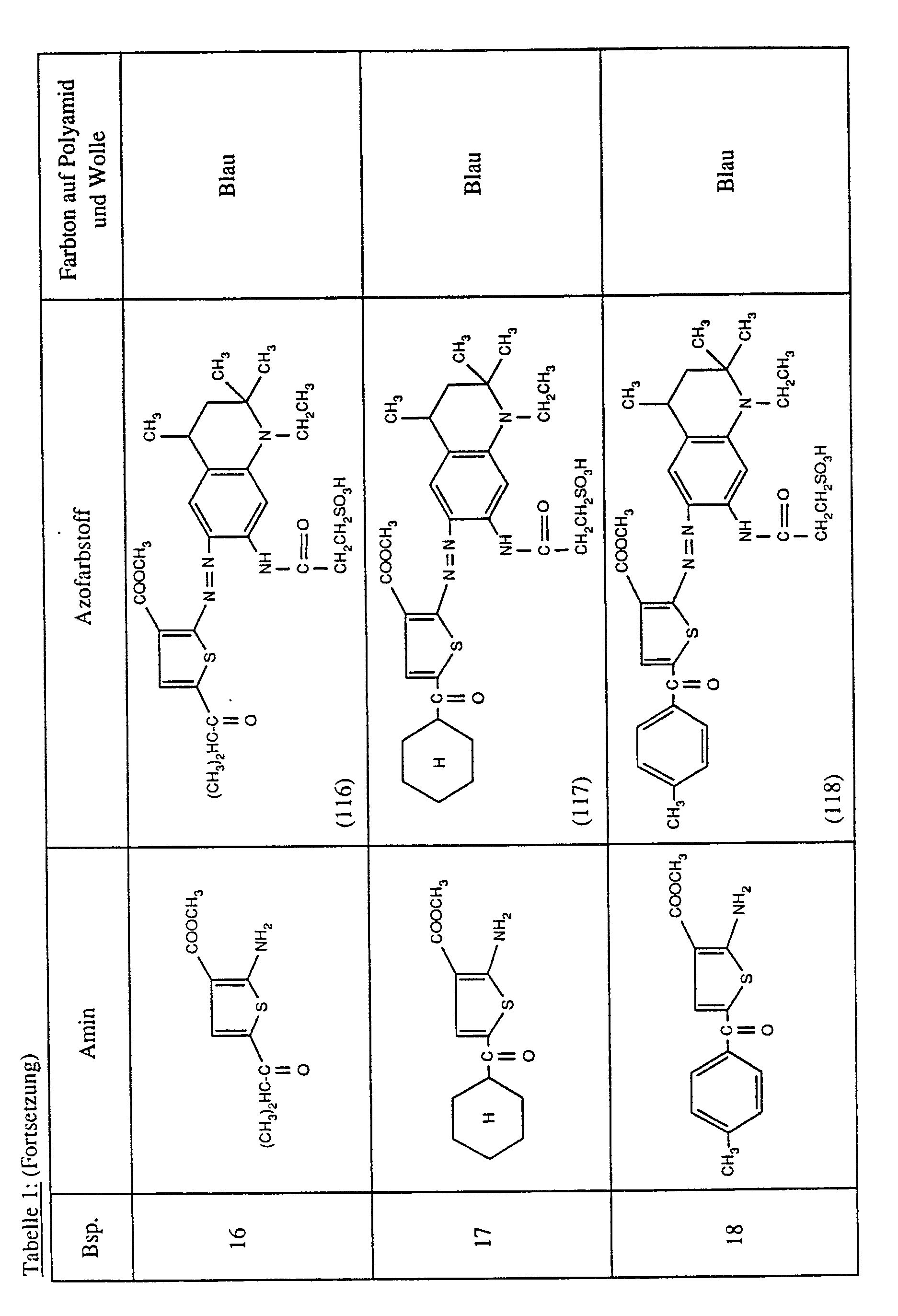

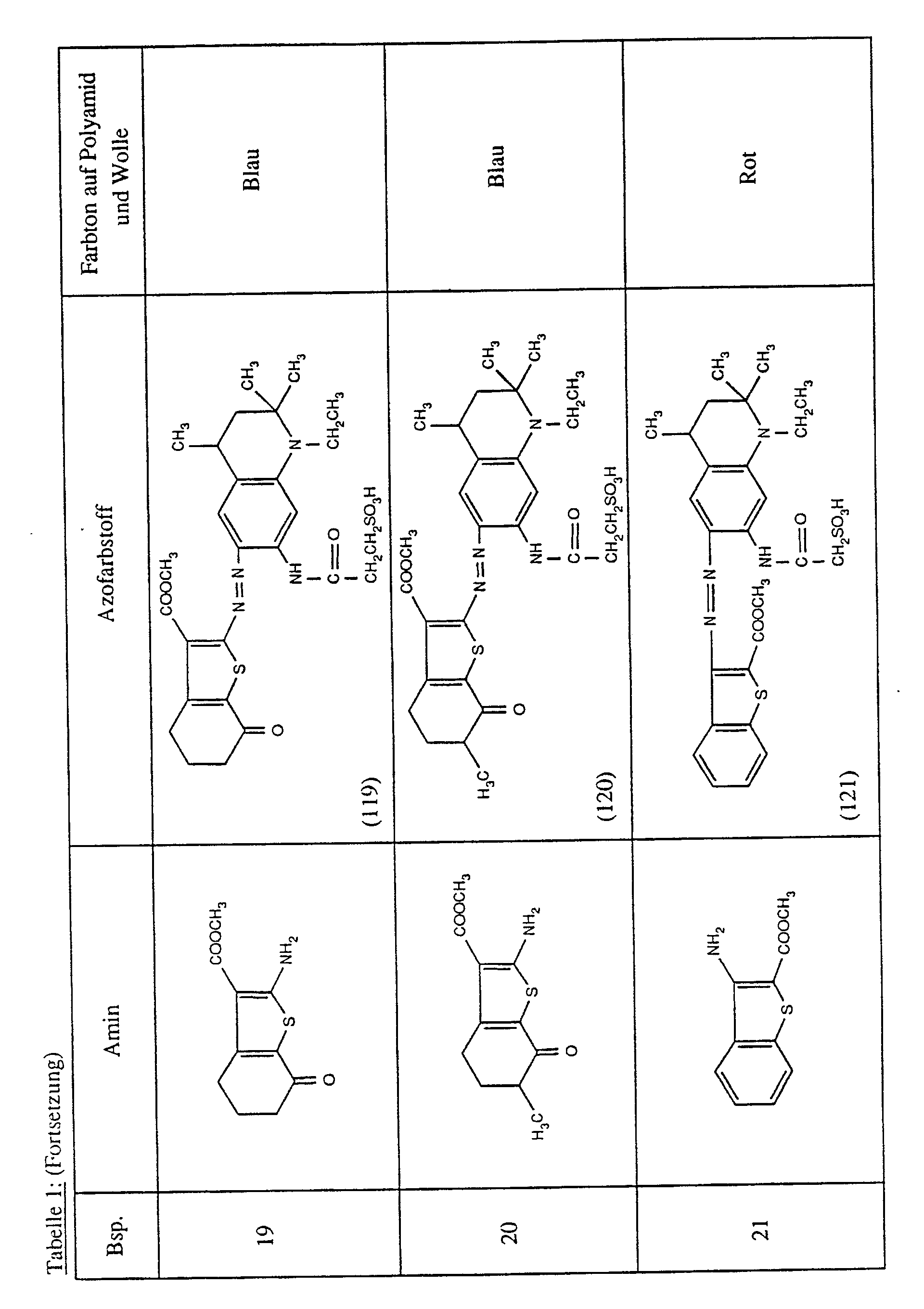

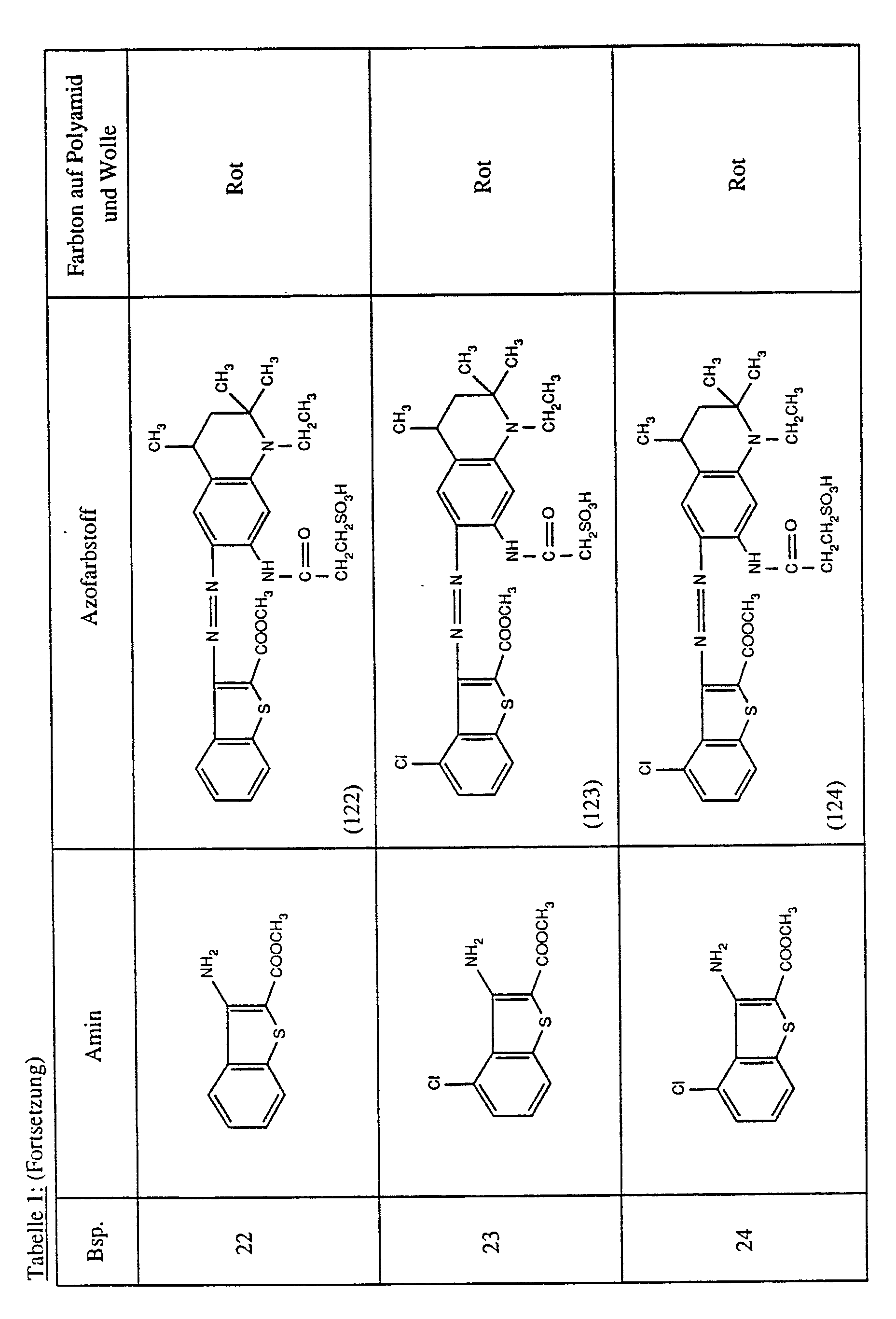

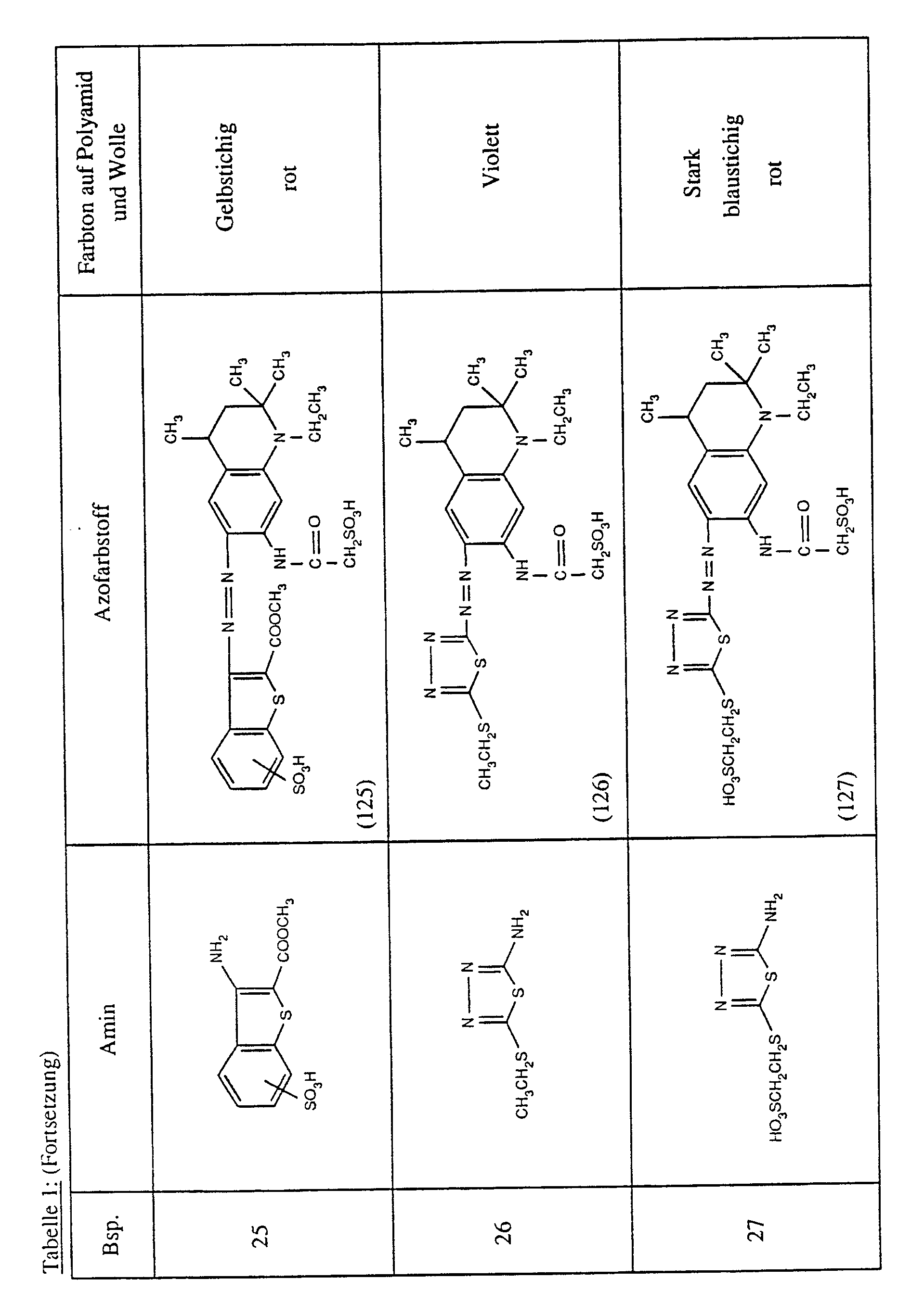

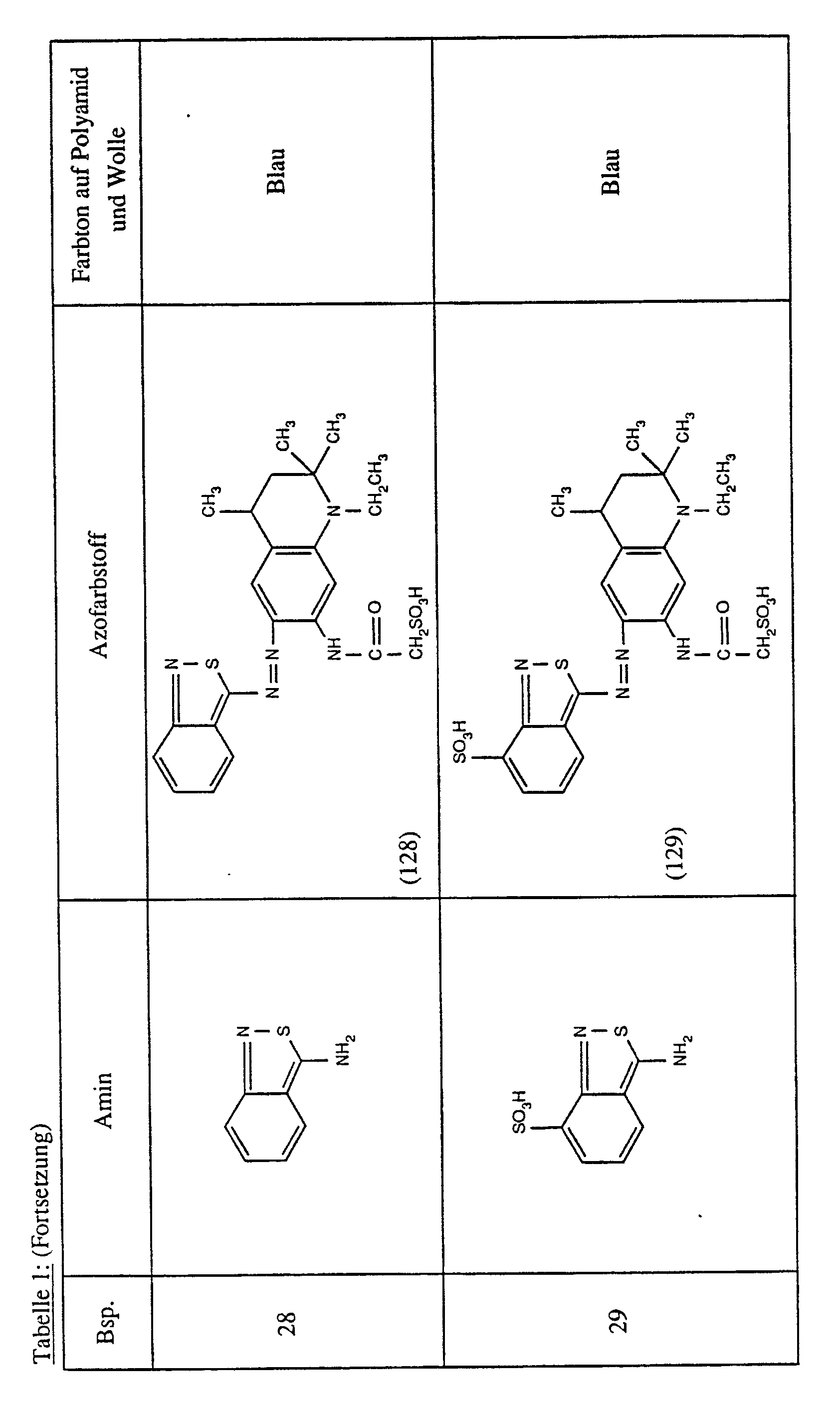

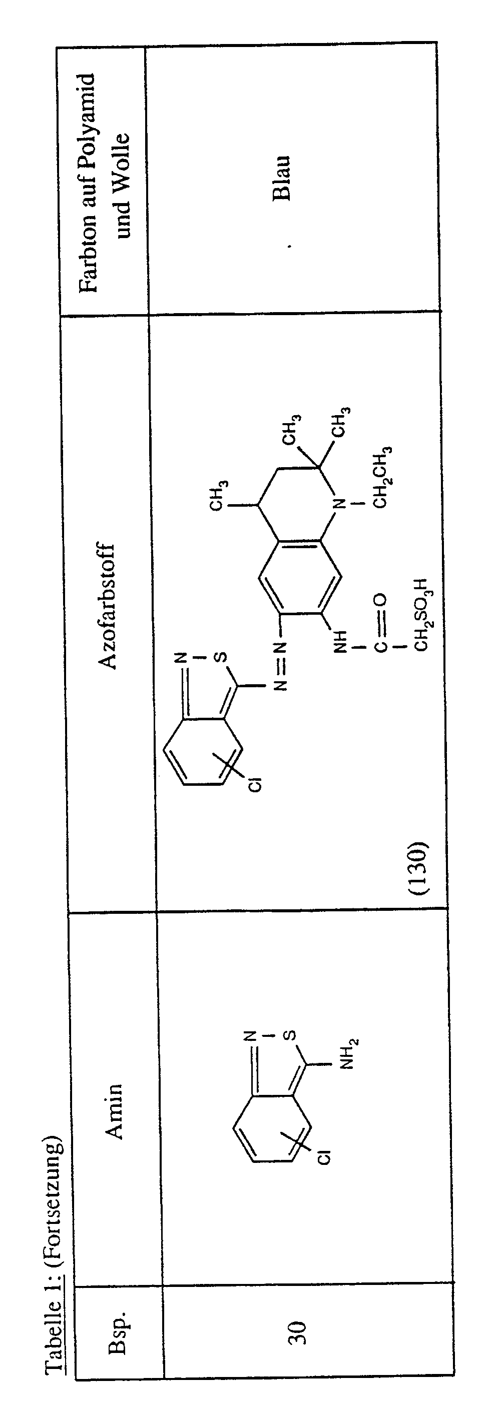

### Beispiel 31:

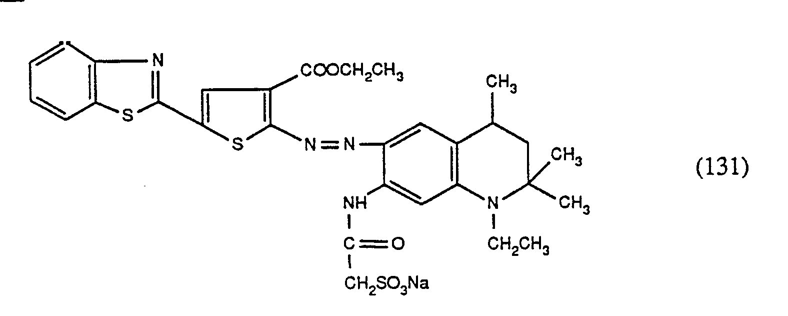

Bei ca. 10° werden 22,5 Teile Essigsäure (100 %), 33 Teile Essigsäure (80 %) und 12 Teile Salzsäure (32 %) unter Rühren vorgelegt. Danach werden 3,2 Teile 2-Amino-5-(benzthiazol-2′-yl)-3-carboäthoxythiophen innerhalb von 2 Minuten portionsweise eingetragen und die Temperatur auf 0° gesenkt. Anschliessend wird eine Lösung aus 0,74 Teilen NaNO₂ in 3 Teilen Wasser zugegeben, bei 0 bis 2° diazotiert und 20 Minuten bei 0° nachgerührt. Nach Zugabe von 1 Teil Sulfaminsäure wird kurz nachgerührt und 3,65 Teile der Kupplungskomponente der Formel (105) bei 0° innerhalb von 1 Minute eingetragen. Anschliessend rührt man 10 Minuten bei 0 bis 2° nach und gibt innerhalb der nächsten 20 Minuten 24 Teile Natriumacetat x 3H₂O in 4 Portionen zu. Nach 30 Minuten Rühren bei 0 bis 2° tropft man eine Lösung von 6 Teilen Natriumacetat, 3 Teilen Soda und 30 Teilen Wasser innerhalb 30 Minuten bei 0 bis 3° zu, entfernt das Eisbad und versetzt die Reaktionsmischung mit 125 Teilen 15%-iger wässriger Natriumchlorid-Lösung. Nach Filtration wird der erhaltene Farbstoff im Vakuum bei 50° getrocknet. Man erhält 8,9 Teile eines schwarzen Pulvers, das der Verbindung der Formel (131) entspricht. Der erhaltene Farbstoff färbt natürliches und synthetisches Polyamidfasermaterial in blauen Farbtönen.

### Beispiele 32 bis 36:

Verfährt man wie in Beispiel 31 angegeben, verwendet jedoch statt 3,2 Teilen 2-Amino-5-(benzthiazol-2′-yl)-3-carboäthoxythiophen eine äquimolare Menge der in Tabelle 2 in Spalte 2 angegebenen Amine und gegebenenfalls statt 3,65 Teilen der Kupplungskomponente der Formel (105) eine äquimolare Menge einer Kupplungskomponente der Formel (107), so erhält man die in Spalte 3 in Form der freien Säuren angegebenen Azofarbstoffe, die natürliches und synthetisches Polyamidfasermaterial in den in Spalte 4 angegebenen Farbtönen färben.

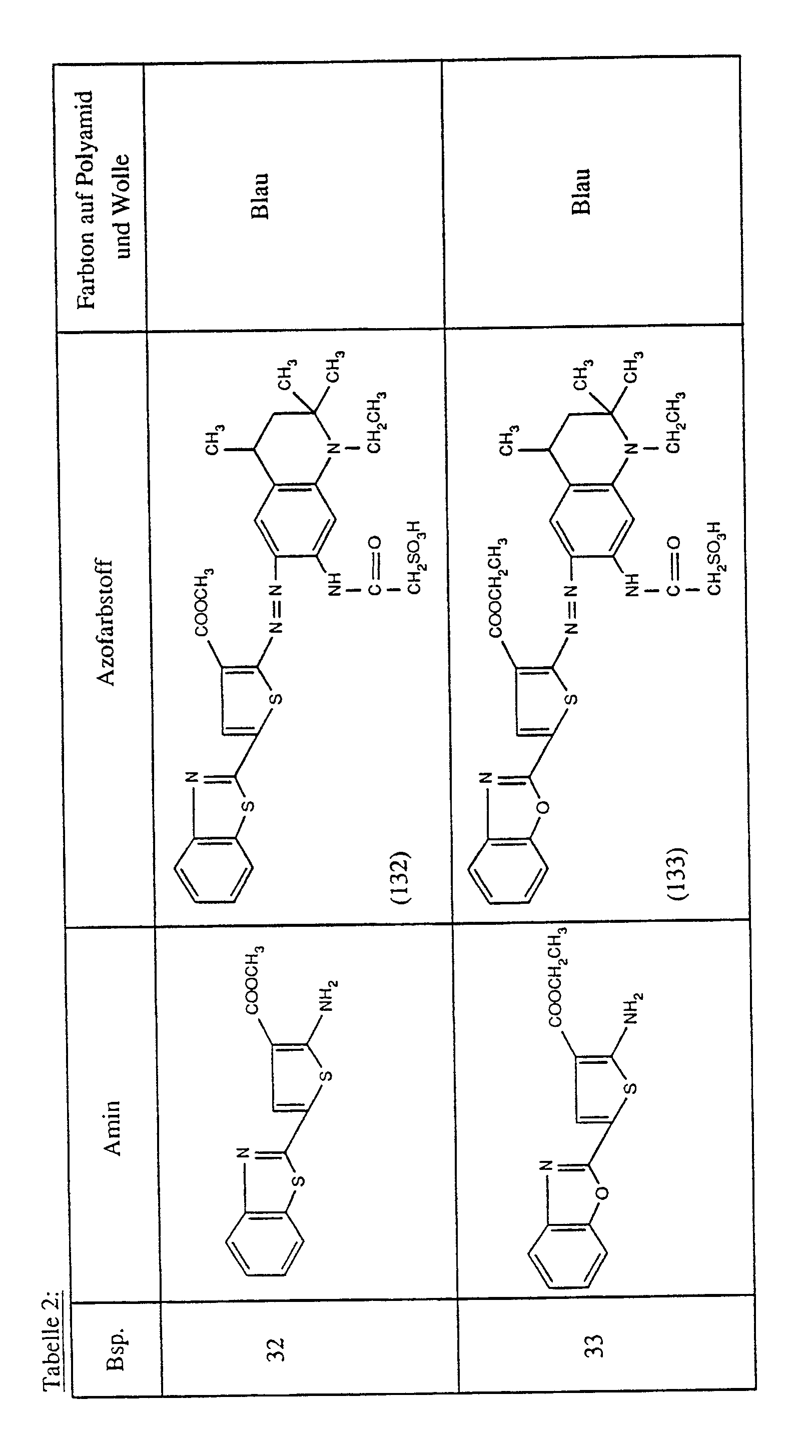

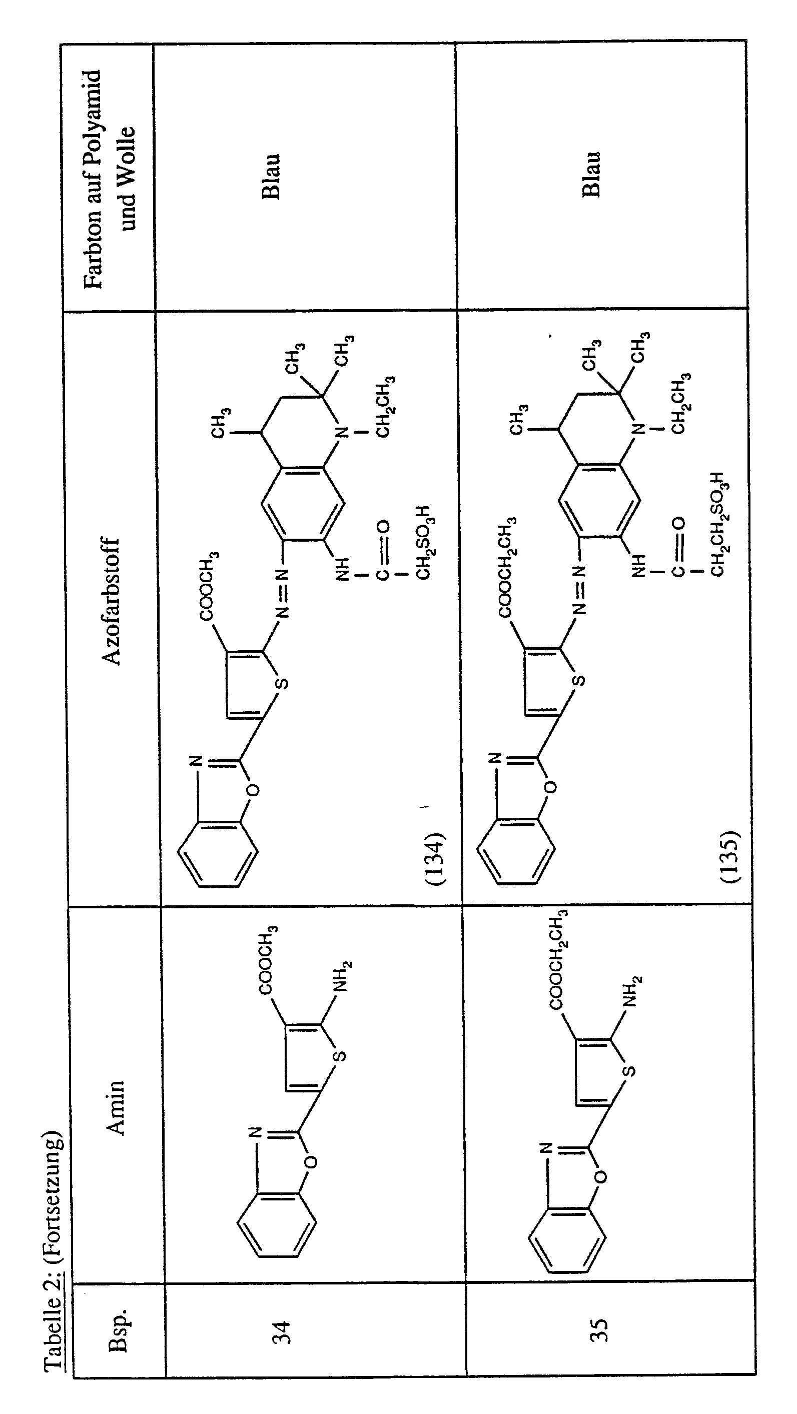

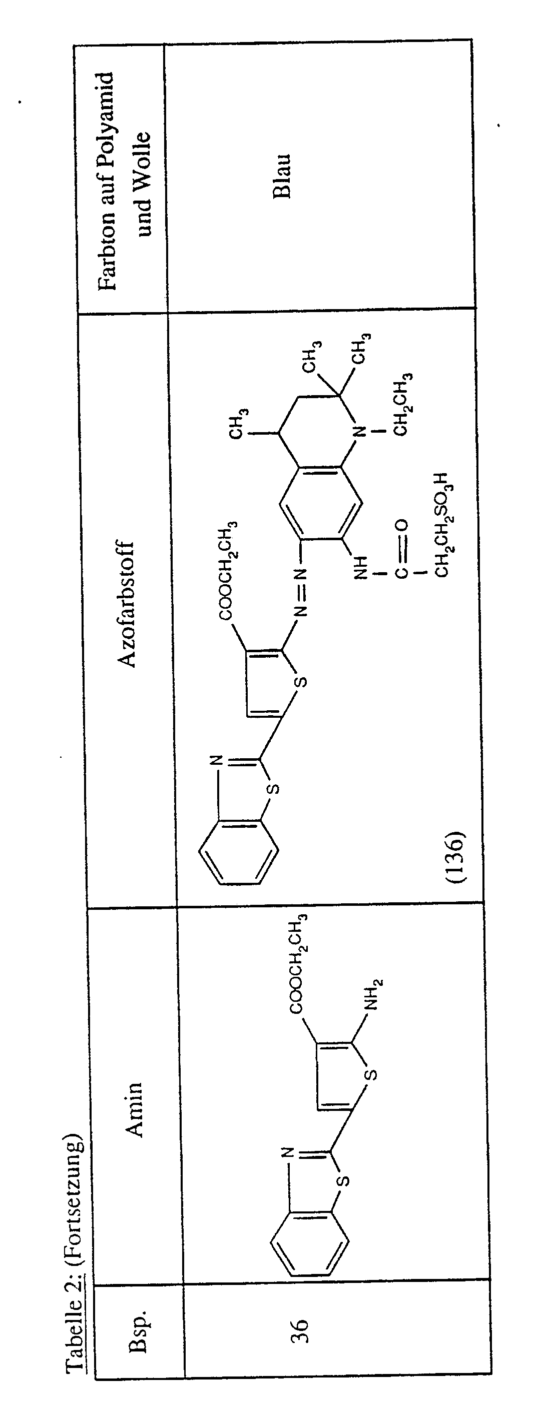

### Beispiel 37:

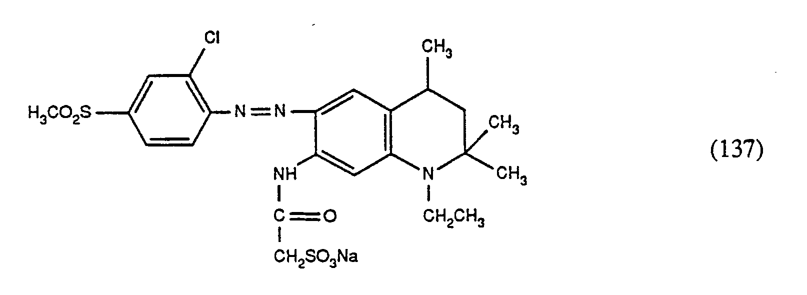

Bei ca. 10° werden 40 Teile Essigsäure (80 %) und 4 Teile Salzsäure (32 %) unter Rühren vorgelegt. Danach werden 2,1 Teile 4-Amino-3-chlorphenylmethylsulfon eingetragen und die Temperatur auf 0° gesenkt. Danach wird innerhalb von 1 Minute eine Lösung aus 0,74 Teilen NaNO₂ in 3 Teilen Wasser zugegeben und bei 0 bis 3° diazotiert und 45 Minuten nachgerührt, wobei die Temperatur 0 bis 3° beträgt. Danach gibt man 1 Teil Sulfaminsäure zu, rührt kurz nach und gibt anschliessend 3,65 Teile der Kupplungskomponente der Formel (105) innerhalb 1 Minute zu. Man rührt 30 Minuten bei 0 bis 3° nach und gibt daraufhin innerhalb 15 Minuten 15 Teile Natriumacetat x 3H₂O in 3 Portionen zu. Nach weiteren 30 Minuten Rühren bei 0 bis 3° wird innerhalb 30 Minuten eine Lösung aus 6 Teilen Natriumacetat, 3 Teilen Soda und 30 Teilen Wasser zugegeben, wobei sich die Reaktionsmischung nicht auf mehr als 20° erwärmt. Anschliessend gibt man innerhalb von 10 Minuten 100 Teile einer 15%-igen wässrigen Natriumchlorid-Lösung zu, filtriert den erhaltenen Farbstoff ab und trocknet im Vakuum bei 50°. Man erhält 6 Teile eines rötlichen Pulvers, das einer Verbindung der Formel (137) entspricht. Der erhaltene Farbstoff färbt natürliches und synthetisches Polyamidfasermaterial in roten Farbtönen.

### Beispiele 38 bis 65:

Verfährt man wie in Beispiel 37 angegeben, verwendet jedoch statt 2,1 Teilen 4-Amino-3-chlorphenylmethylsulfon eine äquimolare Menge der in Tabelle 3 in Spalte 2 angegebenen Amine und gegebenenfalls statt 3,65 Teilen der Kupplungskomponente der Formel (105) eine äquimolare Menge einer Kupplungskomponente der Formel (107), so erhält man die in Spalte 3 in Form der freien Säuren angegebenen Azofarbstoffe, die natürliches und synthetisches Polyamidfasermaterial in den in Spalte 4 angegebenen Farbtönen färben.

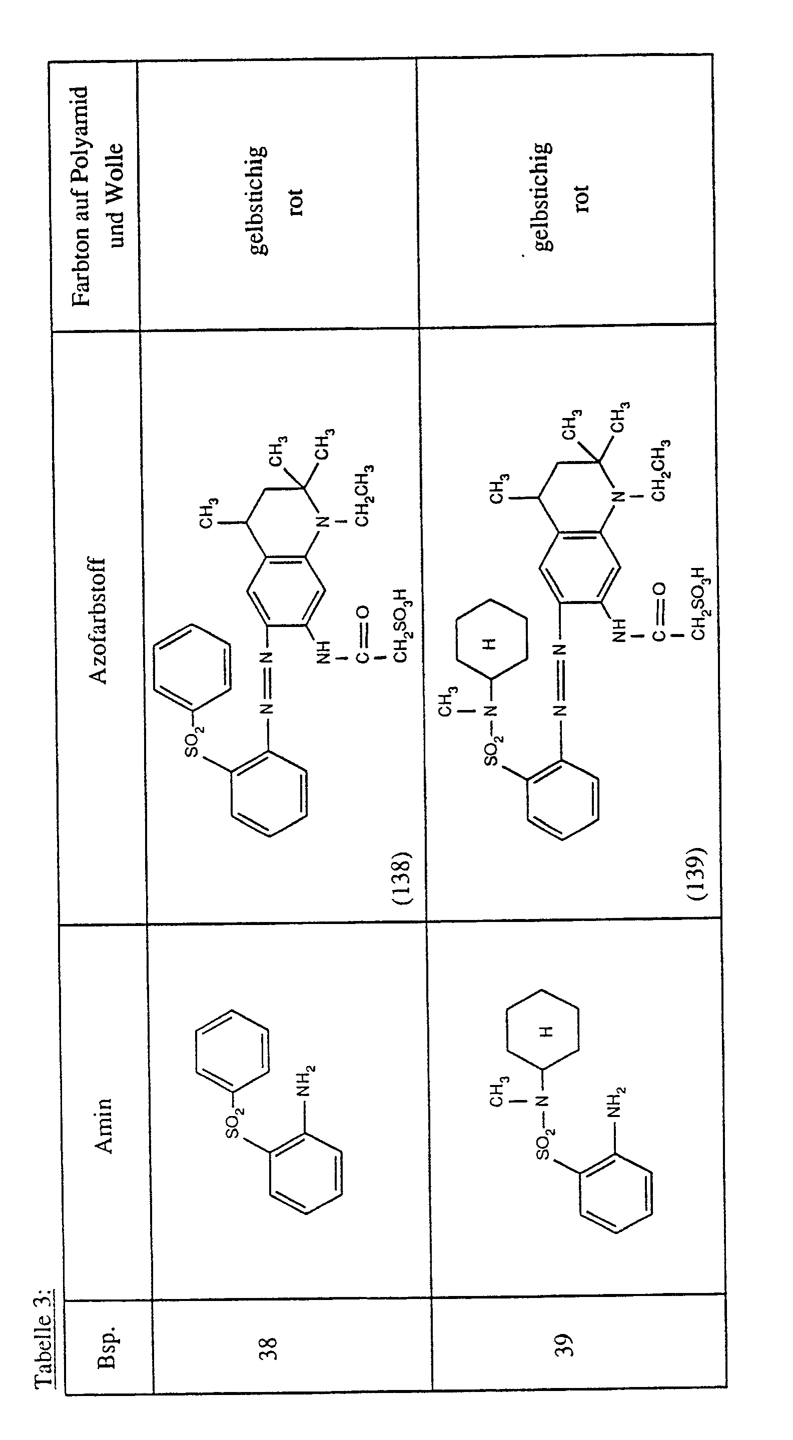

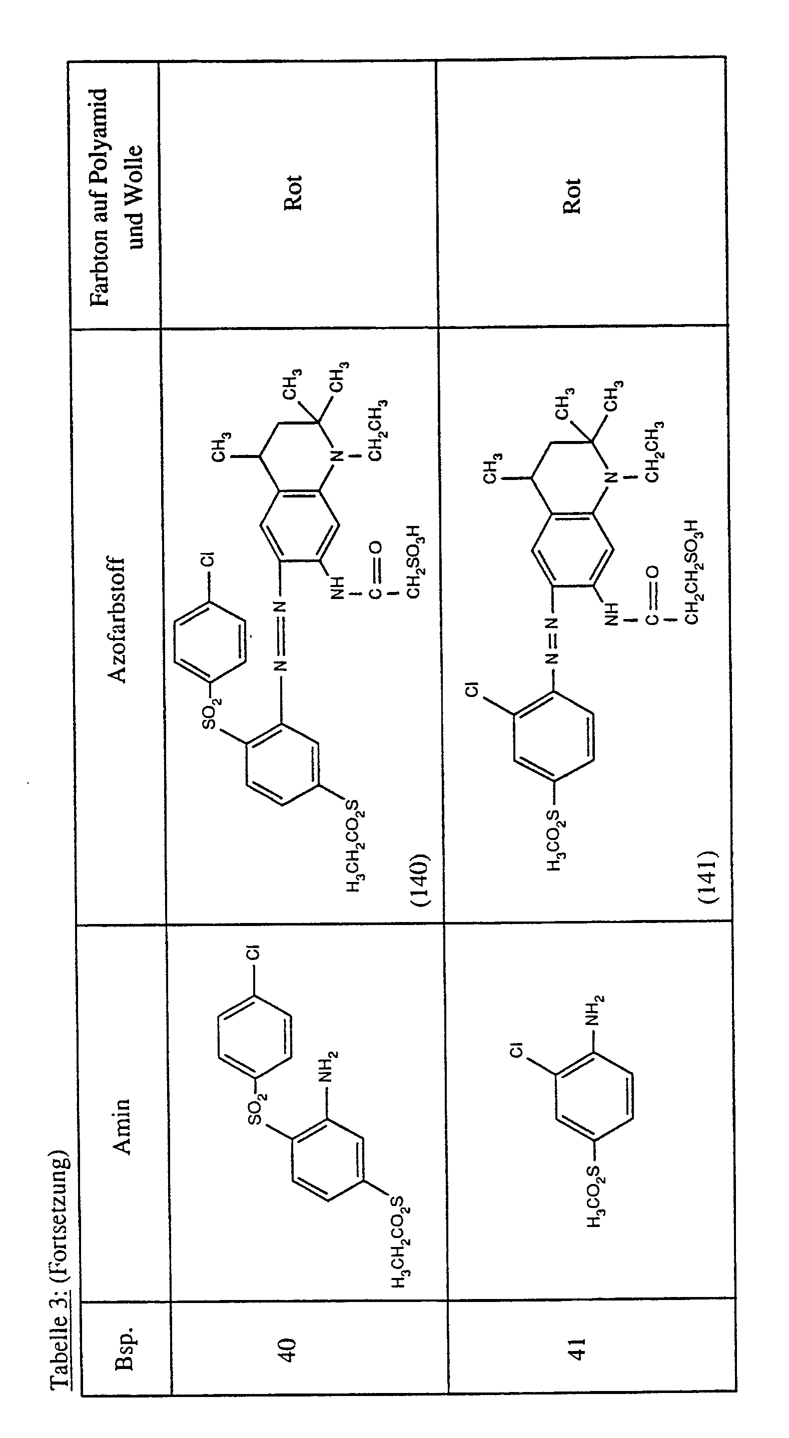

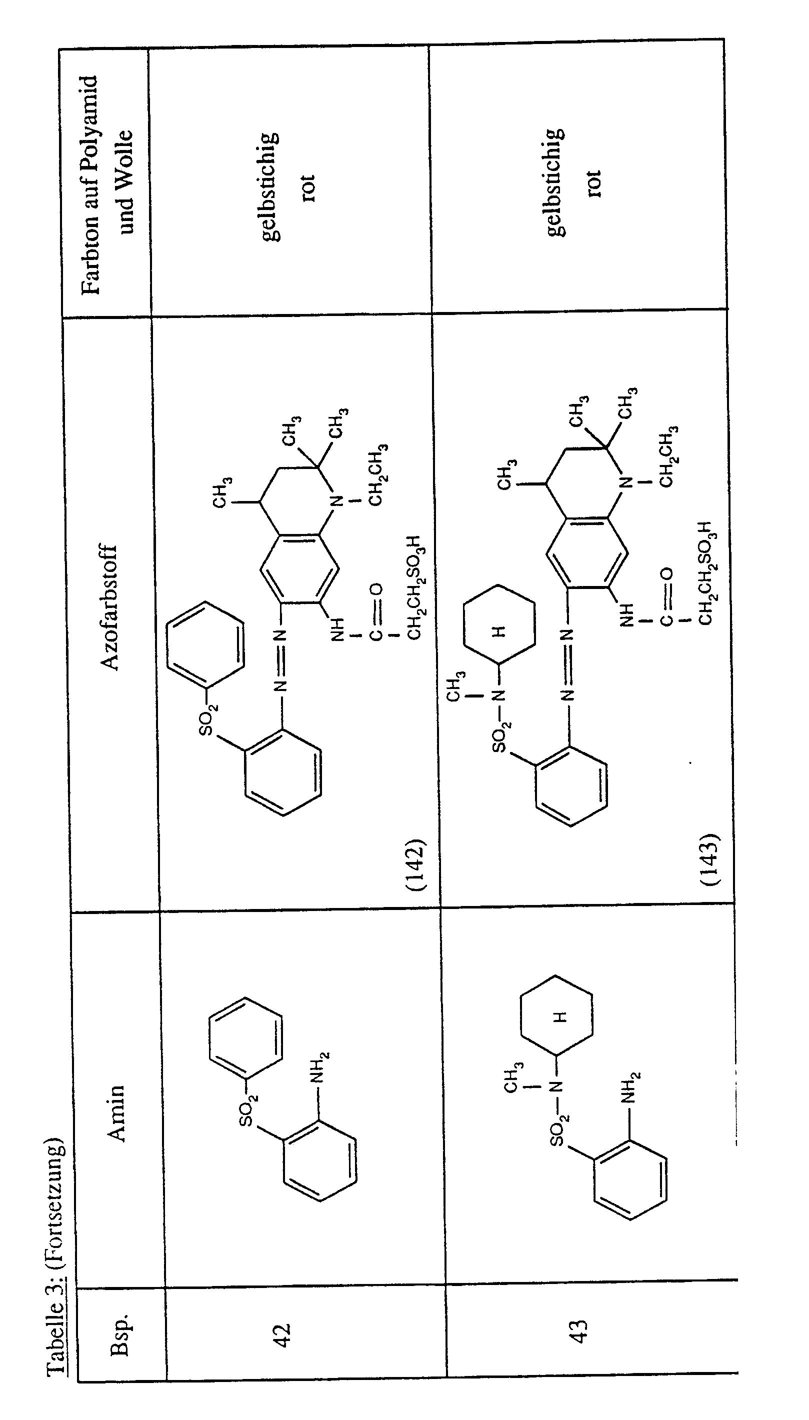

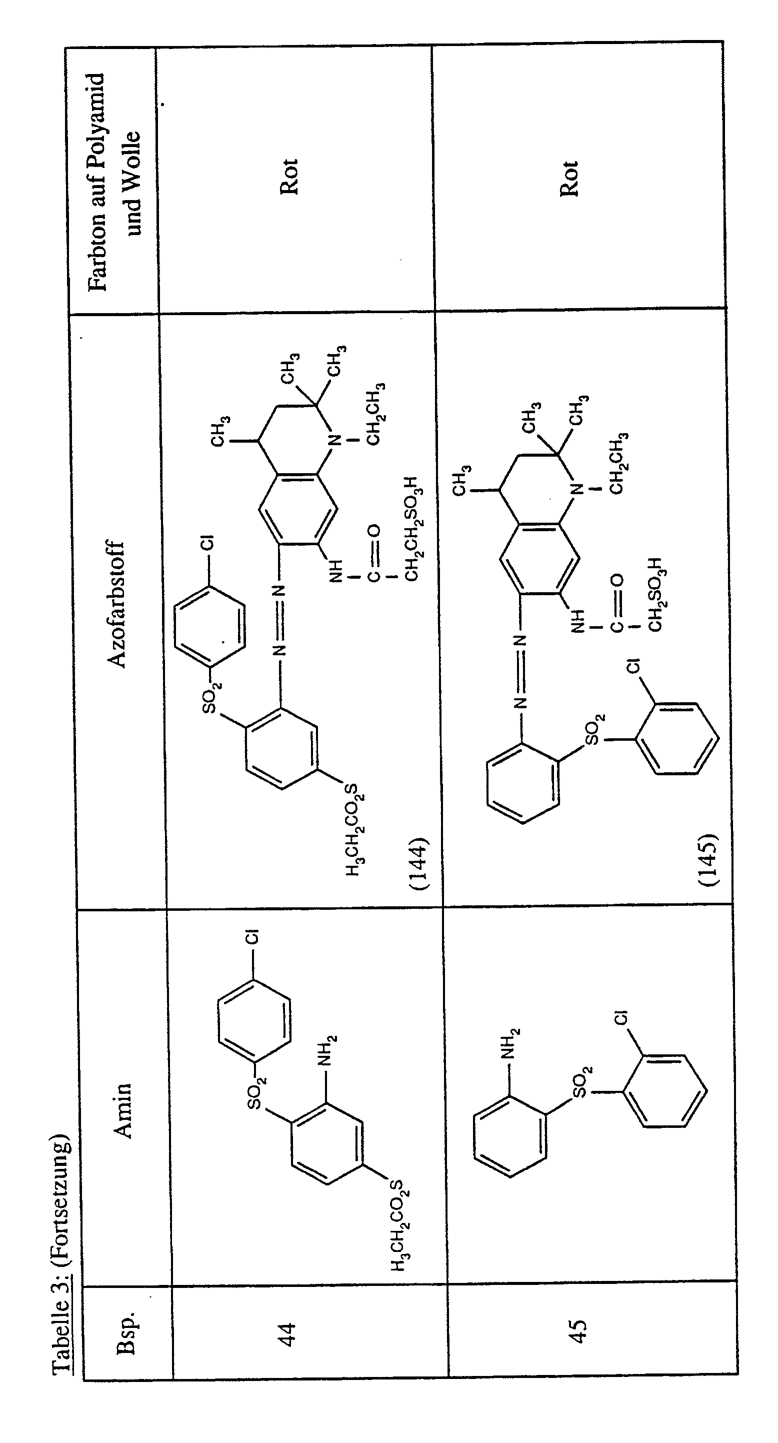

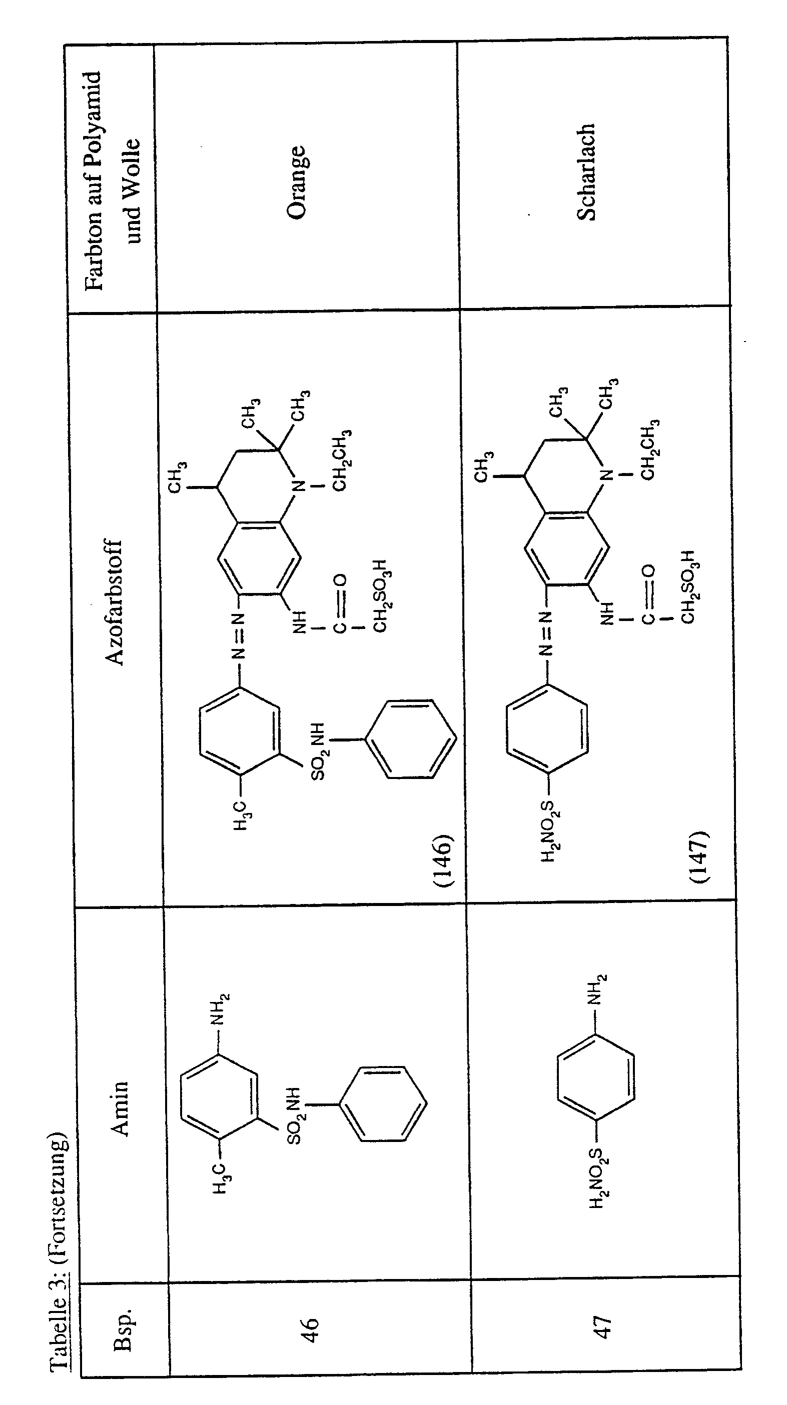

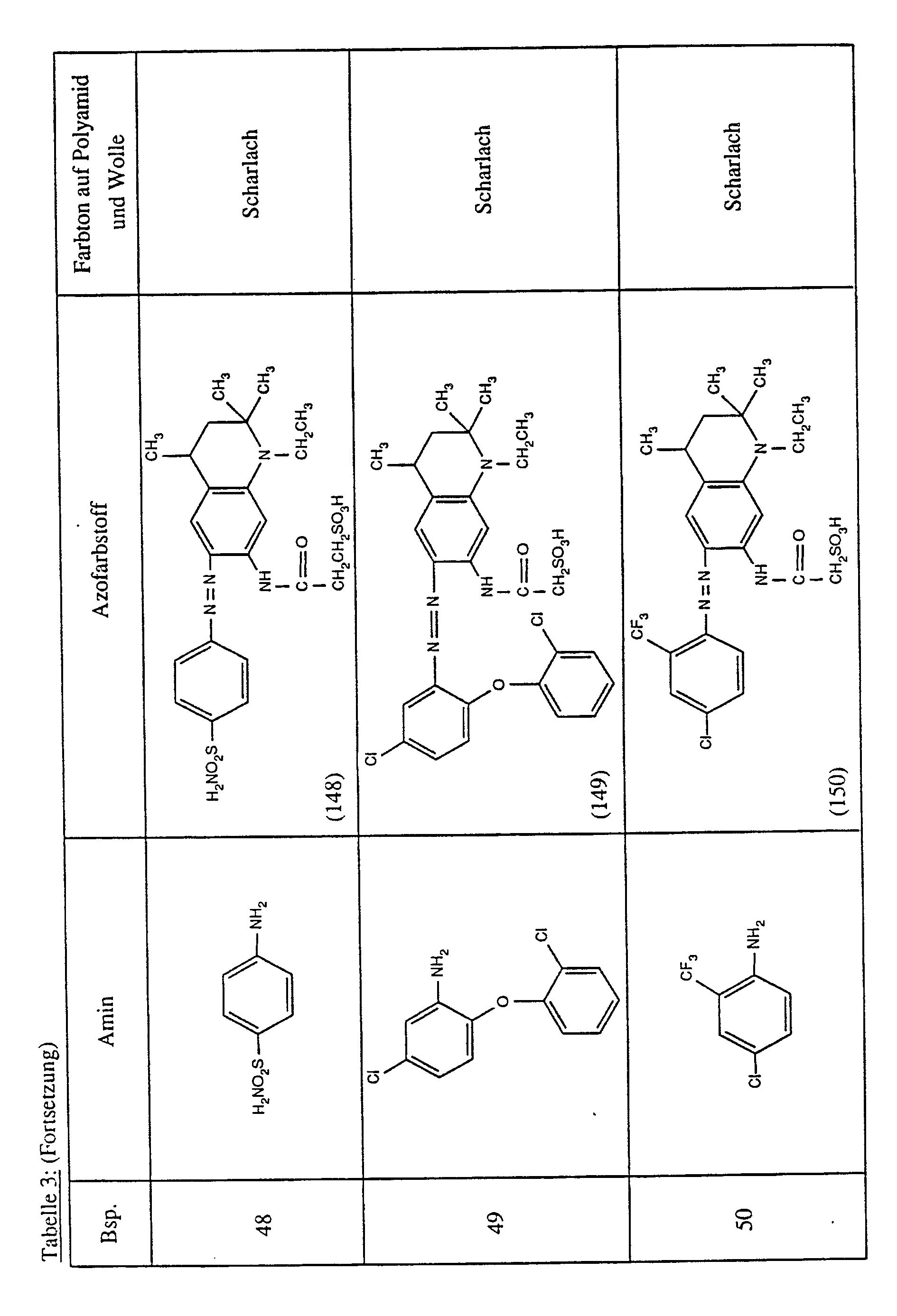

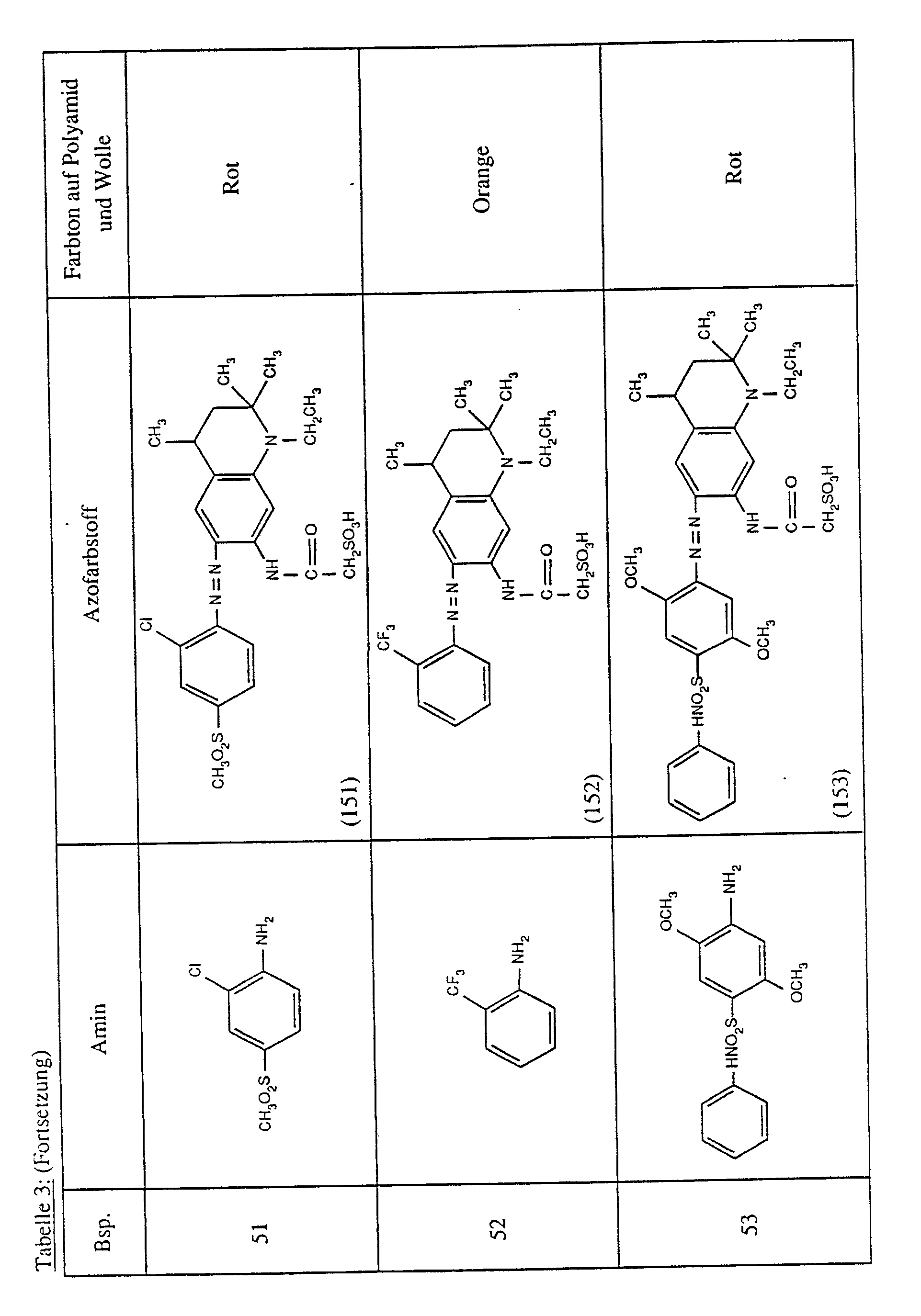

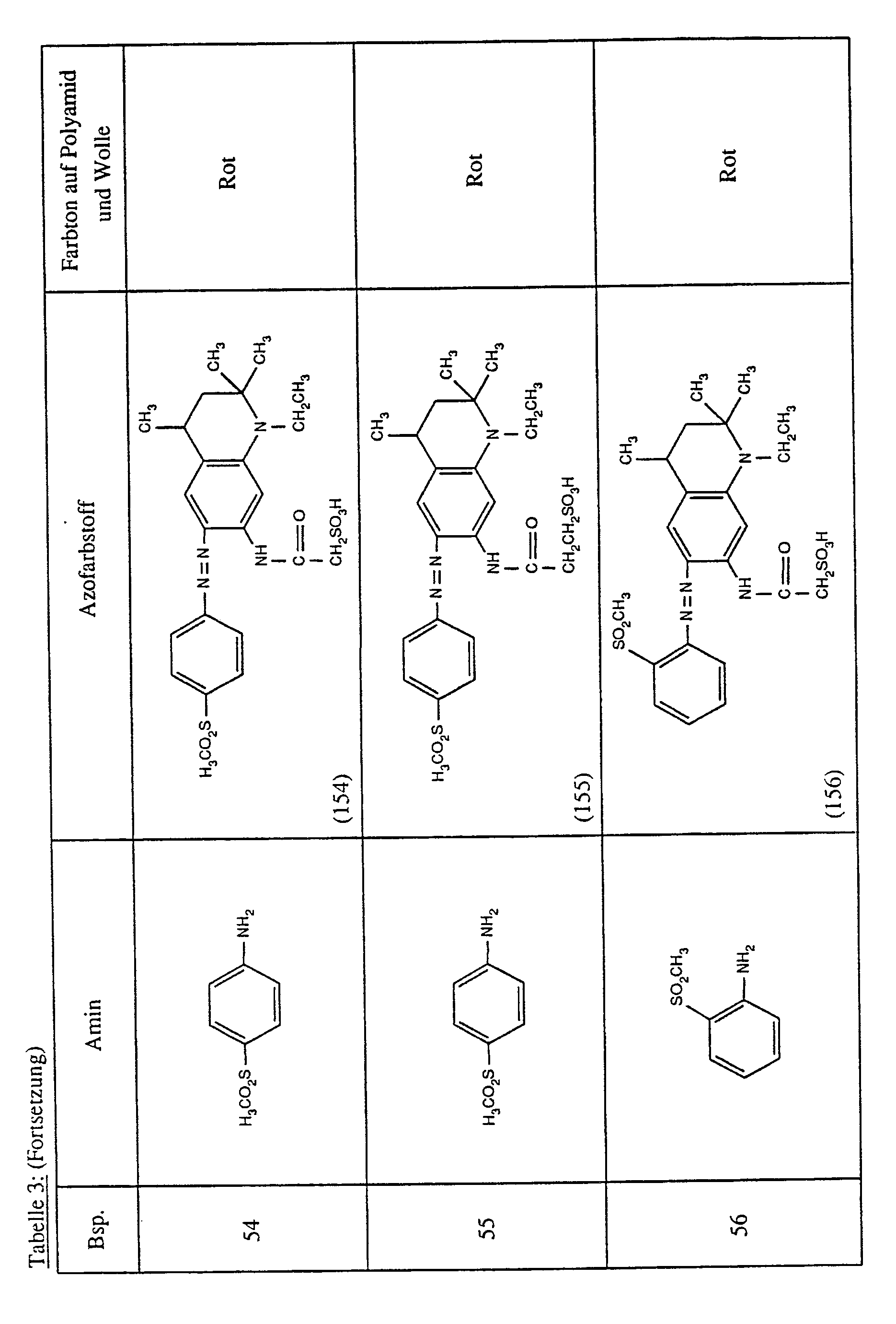

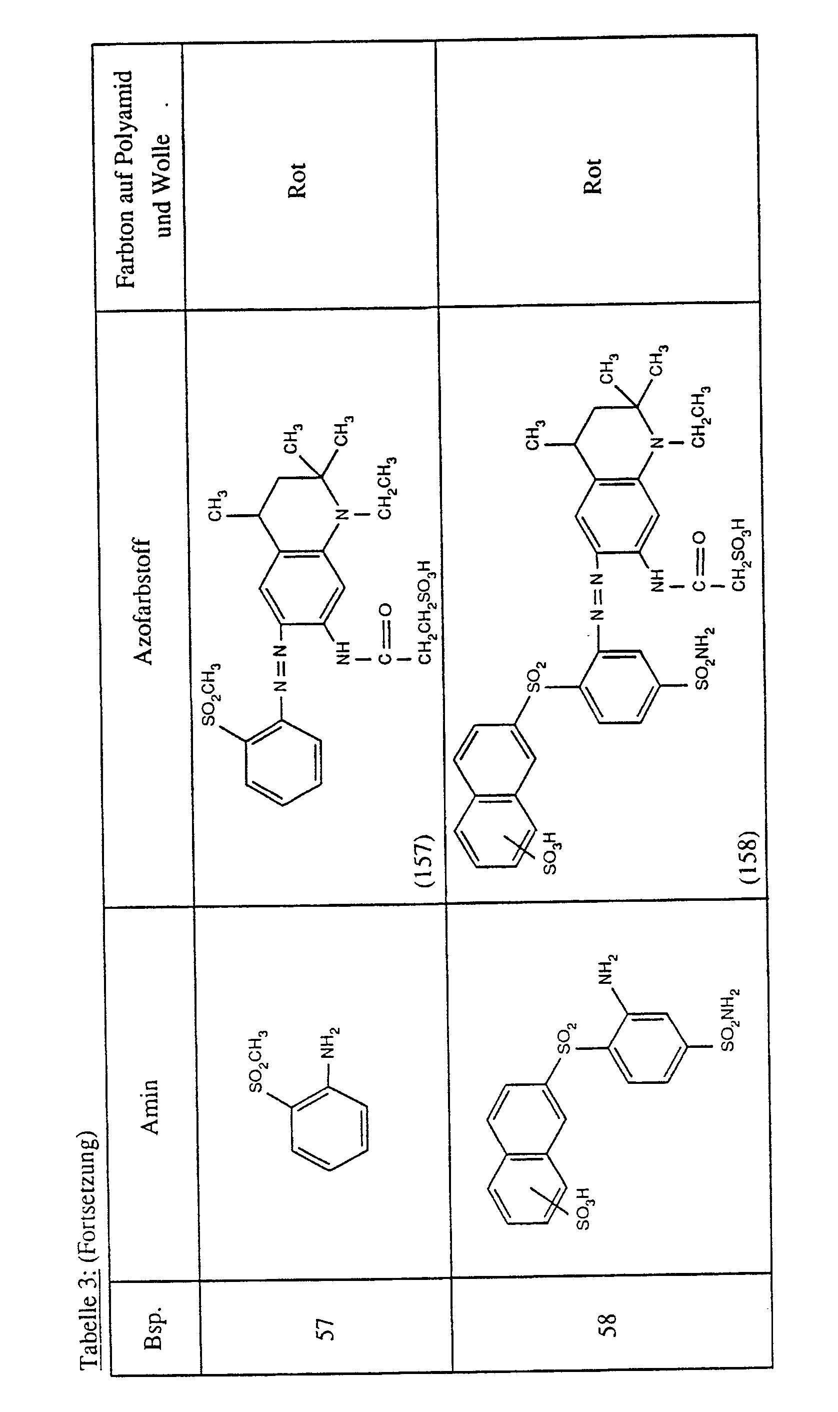

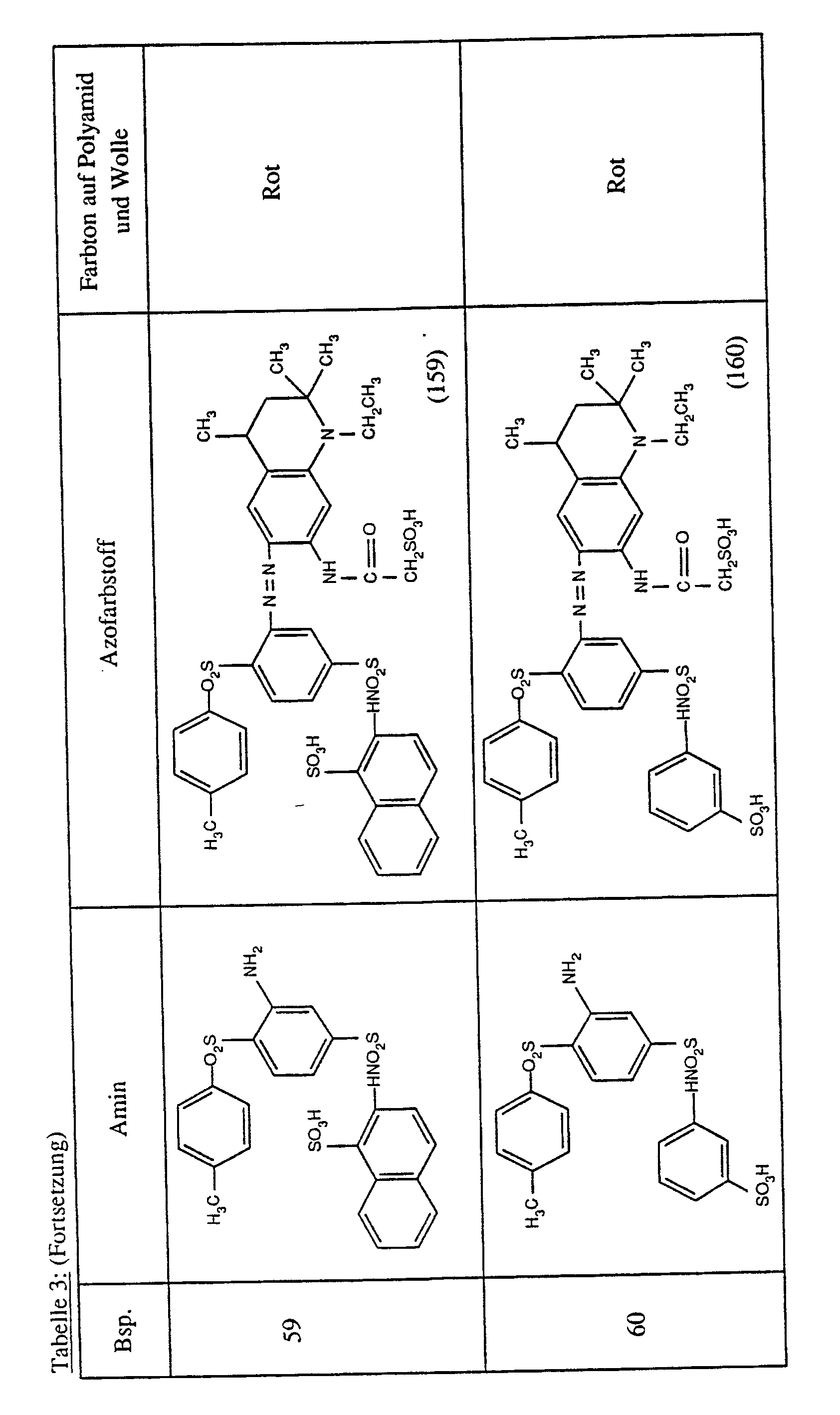

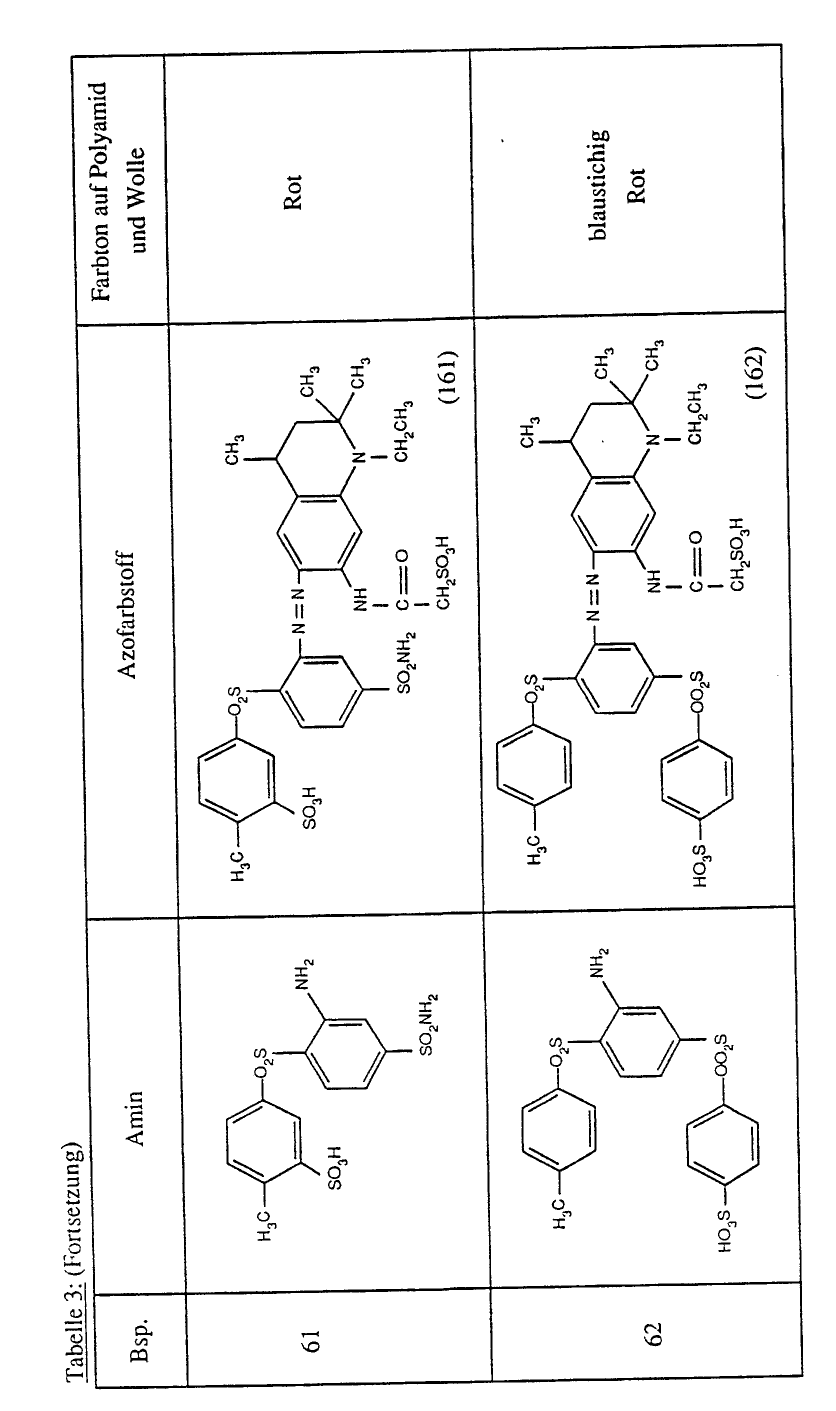

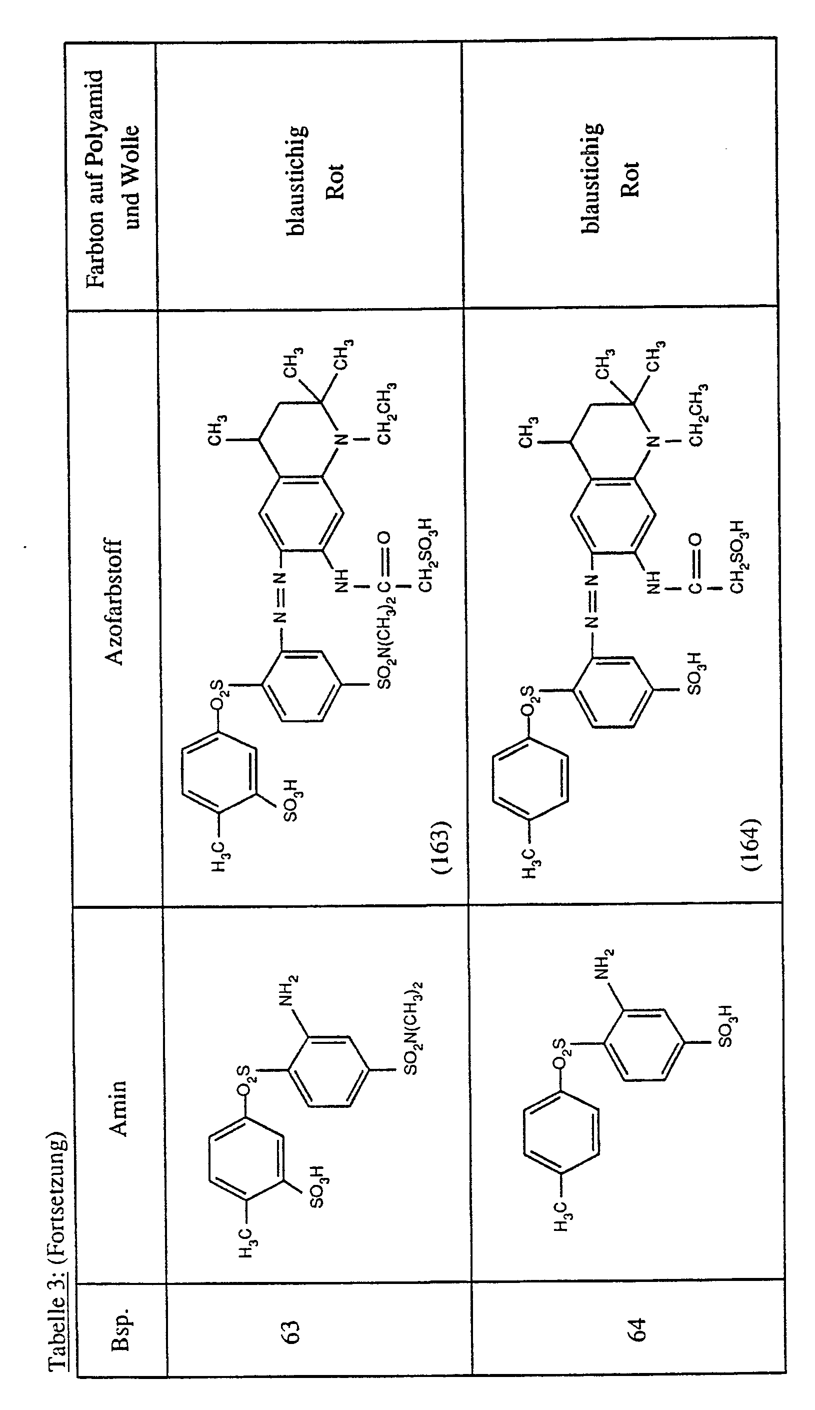

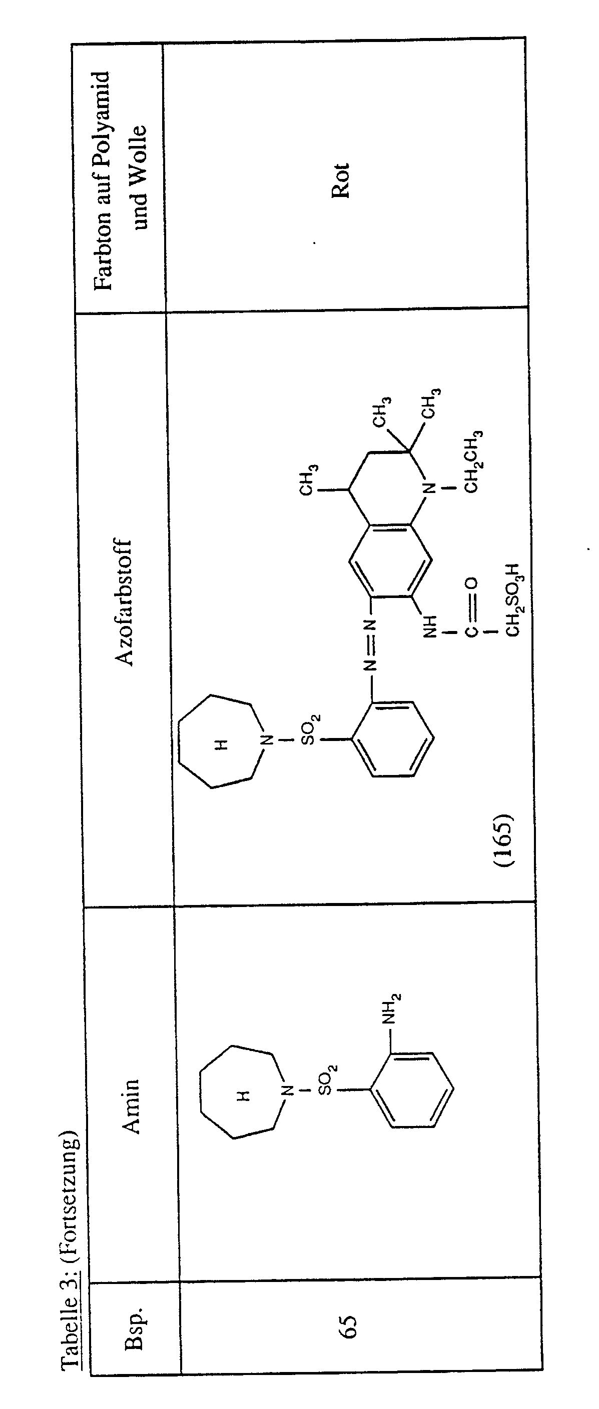

Verfährt man wie in den Beispielen 37 bis 64 angegeben, verwendet jedoch statt einer Kupplungskomponente der Formel (105) bzw. (107) eine Kupplungskomponente der Formel (106) bzw. (108) so erhält man analoge Farbstoffe, die synthetisches und natürliches Polyamidmaterial in den angegebenen Farbtönen färben.

### Beispiel 66:

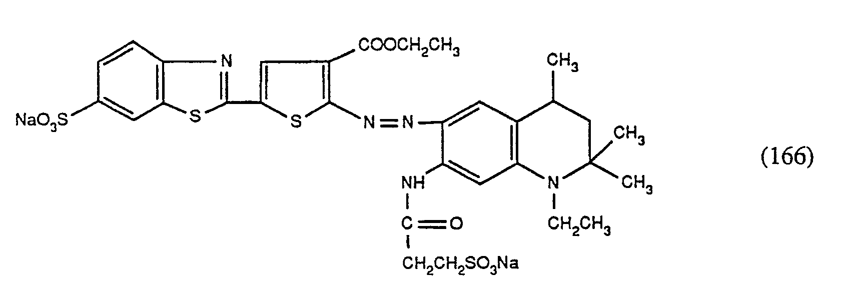

Bei ca. 10° werden 22,5 Teile Essigsäure (100 %), 33 Teile Essigsäure (80 %) und 12 Teile Salzsäure (32 %) unter Rühren vorgelegt. Danach werden 4,27 Teile 2-Amino-5-(2′-benzthiazolyl-6′-sulfonsäure)-3-äthoxycarbonylthiophen innerhalb ca. einer Minute eingetragen und die Temperatur auf 0° gesenkt. Anschliessend tropft man bei 0 bis 2° eine Lösung von 0,74 Teilen Natriumnitrit und 3 Teilen Wasser innerhalb ca. 3 Minuten zu. Es wird 25 Minuten bei 0 bis 2° nachgerührt und danach 1 Teil Sulfaminsäure zugegeben und erneut kurz nachgerührt. Anschliessend werden 3,76 Teile N-Aethyl-7-β-sulfopropionylamido-2,2,4-trimethylchinolin innerhalb ca. 1 Minute eingestreut und die Reaktionsmischung ca. 10 Minuten nachgerührt. Innerhalb 30 Minuten gibt man 24 Teile Natriumacetat x 3 H₂O in 4 Portionen zu und rührt 1 Stunde bei 0 bis 2° nach. Danach wird eine Lösung von 6 Teilen Natriumacetat x 3 H₂O, 3 Teilen Natriumcarbonat und 30 Teilen Wasser zugetropft, 125 Teile einer ca. 25%-igen wässrigen Natriumchlorid-Lösung zugegeben, der Farbstoff abfiltriert und im Vakuum bei 50° getrocknet. Man erhält 8,9 Teile eines Pulvers, das einer Verbindung der Formel (166) entspricht. Der erhaltene Farbstoff färbt synthetisches Polyamidmaterial in blauen Farbtönen.

### Beispiele 67 bis 72:

Wenn man wie in Beispiel 66 angegeben verfährt, jedoch anstatt 4,27 Teilen 2-Amino-5-(2′-Benzthiazolyl-6′-sulfonsäure)-3-äthoxycarbonylthiophen eine äquimolare Menge eines der in der folgenden Tabelle 4 angegebenen Amine der Formel 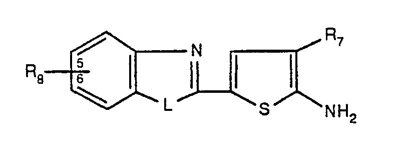 verwendet, worin L, R₇ und R₈ die in der folgenden Tabelle 4 in den Spalten 2, 3 und 4 angegebenen Bedeutungen haben, und anstatt 3,76 Teilen N-Aethyl-7-ß-sulfopropionylamido-2.2.4-trimethylchinolin eine äquimolare Menge einer der in der folgenden Tabelle 4 angegebenen Kupplungskomponenten der Formel 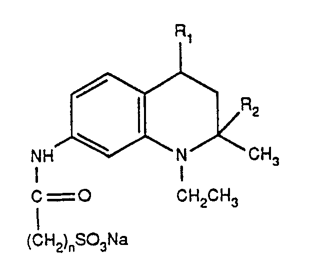 verwendet, worin R₁, R₂ und n die in der folgenden Tabelle 4 in den Spalten 5,6 und 7 angegebenen Bedeutungen haben, so erhält man Azofarbstoffe, die synthetisches Polyamid in den in Spalte 8 angegebenen Farbtönen färben.

**Tabelle 4:**

| Bsp. | L | R₇ | R₈ | R₁ | R₂ | n | Nuance auf Polyamid und Wolle |
|---|---|---|---|---|---|---|---|
| 67 | -S- | -COOCH₂CH₃ | 6-SO₃Na | -CH₃ | -CH₃ | 1 | blau |
| 68 | -S- | -CONH₂ | 6-SO₃Na | -CH₃ | -CH₃ | 1 | blau |
| 69 | -O- | -CONH₂ | 5-SO₃Na | -CH₃ | -CH₃ | 1 | blau |
| 70 | -O- | -CONH₂ | 6-SO₃Na | -CH₃ | -CH₃ | 1 | blau |
| 71 | -S- | -CONH₂ | 5-SO₃Na | -H | -H | 1 | blau |
| 72 | -S- | -CONH₂ | 6-SO₃Na | -H | -H | 1 | blau |
| 73 | -O- | -CONH₂ | 5-SO₃Na | -H | -H | 1 | blau |
| 74 | -O- | -CONH₂ | 6-SO₃Na | -H | -H | 1 | blau |
| 75 | -S- | -CONH₂ | 6-SO₃Na | -CH₃ | -CH₃ | 2 | blau |
| 76 | -O- | -CONH₂ | 5-SO₃Na | -CH₃ | -CH₃ | 2 | blau |
| 77 | -O- | -CONH₂ | 6-SO₃Na | -CH₃ | -CH₃ | 2 | blau |
| 78 | -S- | -COOCH₂CH₃ | 5-SO₃Na | -CH₃ | -CH₃ | 2 | blau |
| 79 | -S- | -COOCH₂CH₃ | 6-SO₃Na | -H | -H | 2 | blau |
| 80 | -O- | -COOCH₂CH₃ | 5-SO₃Na | -H | -H | 2 | blau |
| 81 | -O- | -COOCH₂CH₃ | 6-SO₃Na | -H | -H | 2 | blau |
| 82 | -S- | -COOCH₂CH₃ | 6-SO₃Na | -H | -H | 1 | blau |

### Färbevorschrift 1:

Man färbt 10 Teile Polyamid-6,6-Gewebe in 500 Teilen einer wässrigen Flotte, die 2 g/l Ammonacetat enthält und mit Essigsäure auf pH 5 gestellt wird. Der Anteil des Farbstoffes gemäss Beispiel 9 beträgt 0,7 % bezogen auf das Fasergewicht. Die Färbedauer bei einer Temperatur von 98° beträgt 30 bis 90 Minuten. Das gefärbte Polyamid-6,6-Gewebe wird anschliessend herausgenommen und wie üblich gewaschen und getrocknet.

Man erhält ein blau gefärbtes Polyamid-6,6-Gewebe, das eine reine Nuance und gute Gesamtechtheiten aufweist.

### Färbevorschrift II:

Man färbt 10 Teile Polyamid-6,6-Gewebe in 500 Teilen einer wässrigen Flotte, die 1 g/l Mononatriumphosphat enthält und mit Dinatriumphosphat auf pH 6 gestellt wird. Der Anteil des Farbstoffes gemäss Beispiel 31 beträgt 1 %, bezogen auf das Fasergewicht. Die Färbedauer bei einer Temperatur von 98° beträgt 30 bis 90 Minuten. Das gefärbte Polyamid-6,6-Gewebe wird anschliessend herausgenommen und wie üblich gewaschen und getrocknet.

Man erhält ein blau gefärbtes Polyamid-6,6-Gewebe, das eine reine Nuance und gute Gesamtechtheiten aufweist.

### Färbevorschrift III:

Man färbt 10 Teile Wollstück in 500 Teilen einer wässrigen Flotte. Bezogen auf das Fasergewicht betragen die Anteile an Farbstoff 0,45 gemäss Beispiel 31, an Glaubersalz kalz. 5 % und 80%-iger Essigsäure 2 %. Die Färbedauer bei einer Temperatur von 98° beträgt 30-60 Minuten. Das blau gefärbte, wie üblich gewaschene und getrocknete Wollstück weist sehr gute Allgemeinechtheiten auf.

## Patentansprüche

1. Azofarbstoffe der Formel 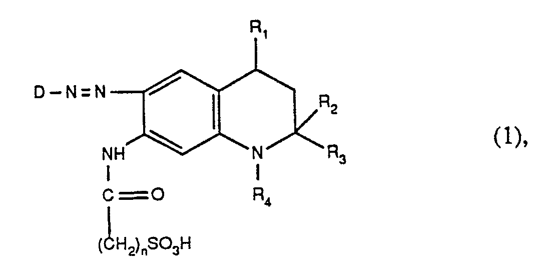 worin D eine Diazokomponente, R₁, R₂, R₃ und R₄ unabhängig voneinander Wasserstoff oder gegebenenfalls substituiertes C₁-C₈-Alkyl und n die Zahl 1, 2, 3, 4 oder 5 ist.

2. Azofarbstoffe gemäss Anspruch 1, worin D eine Diazokomponente der Benzol- oder Naphthalinreihe oder der heterocyclischen Reihe ist.

3. Azofarbstoffe gemäss Anspruch 1, worin D Thienyl, Thiazolyl, Isothiazolyl, 1,2,4-Thiadiazolyl, 1,3,4-Thiadiazolyl, Benzthiazolyl, Benzisothiazolyl, Benzoxazolyl, Pyrazolyl, Imidazolyl, 1,2,3-Triazolyl, 1,2,4-Triazolyl, Thiophenyl, Benzthiophenyl, Tetrahydrobenzthiophenyl, 7-Oxotetrahydrobenzo[b]thiophenyl, Pyridinyl, Indazolyl, Phenyl oder Naphthyl ist, wobei jeder der genannten Reste substituiert sein kann durch C₁-C₈-Alkyl, C₁-C₈-Alkoxy, C₂-C₈-Alkanoylamino, C₂-C₈-Alkoxycarbonylamino, C₂-C₈-Alkanoyl, C₅-C₇-Cycloalkylcarbonyl, im Cycloalkylring durch C₁-C₄-Alkyl, Halogen, Sulfo oder Sulfato substituiertes C₅-C₇-Cycloalkylcarbonyl, Benzoyl, im Phenylring durch C₁-C₄-Alkyl, Halogen, Sulfo oder Sulfato substituiertes Benzoyl, C₁-C₈-Alkylthio, durch Sulfo substituiertes C₁-C₈-Alkylthio, Benzthiazol, Benzoxazol, durch C₁-C₄-Alkyl, Halogen, Sulfo oder Sulfato substituiertes Benzthiazol oder Benzoxazol, Benzoylamino, Amino, Mono- oder Dialkylamino mit 1 bis 8 Kohlenstoffatomen im Alkylrest, Phenylamino, C₂-C₈-Alkoxycarbonyl, Nitro, Cyan, Trifluormethyl, Halogen, 1-Azacycloheptan-N-sulfonyl, Sulfamoyl, am Stickstoffatom ein- oder zweifach durch C₁-C₄-Alkyl, C₅-C₇-Cycloalkyl, Phenyl oder Naphthyl substituiertes Sulfamoyl, im Phenyl- bzw. Naphthylring durch C₁-C₄-Alkyl, Halogen, Sulfo oder Sulfato substituiertes Phenyl- oder Naphthylaminosulfonyl, Phenoxy, Phenoxysulfonyl, durch C₁-C₄-Alkyl, Halogen, Sulfo oder Sulfato substituiertes Phenoxy oder Phenoxysulfonyl, Carbamoyl, Ureido, Hydroxy, C₁-C₈-Alkylsulfonyl, Phenylsulfonyl, Naphthylsulfonyl, im Phenylring bzw. Naphthylring durch C₁-C₄-Alkyl, Halogen, Sulfo oder Sulfato substituiertes Phenylsulfonyl oder Naphthylsulfonyl, Carboxy, Sulfomethyl, Sulfo, Sulfato, Thiosulfato und Phenylazo- und Naphthylazogruppen, und gegebenenfalls je 2 benachbarte Substituenten der genannten Ringsysteme weitere ankondensierte Phenylringe oder Cyclohexylringe bilden.

4. Azofarbstoffe gemäss Anspruch 1, worin D Thienyl, Thiazolyl, Isothiazolyl, 1,2,4-Thiadiazolyl, 1,3,4-Thiadiazolyl, Benzthiazolyl, Benzisothiazolyl, Benzoxazolyl, Pyrazolyl, Imidazolyl, 1,2,3-Triazolyl, 1,2,4-Triazolyl, Thiophenyl, Benzthiophenyl, Tetrahydrobenzthiophenyl, 7-Oxo-tetrahydrobenzo[b]thiophenyl, Pyridinyl, Indazolyl, Phenyl oder Naphthyl ist, wobei jeder der genannten Reste substituiert sein kann durch C₁-C₈-Alkyl, C₁-C₈-Alkoxy, C₂-C₈-Alkanoylamino, C₂-C₈-Alkoxycarbonylamino, C₂-C₈-Alkanoyl, C₅-C₇-Cycloalkylcarbonyl, im Cycloalkylring durch C₁-C₄-Alkyl, Halogen, Sulfo oder Sulfato substituiertes C₅-C₇-Cycloalkylcarbonyl, Benzoyl, im Phenylring durch C₁-C₄-Alkyl, Halogen, Sulfo oder Sulfato substituiertes Benzoyl, Benzthiazol, Benzoxazol, durch C₁-C₄-Alkyl, Halogen, Sulfo oder Sulfato substituiertes Benzthiazol oder Benzoxazol, Benzoylamino, Amino, Mono- oder Dialkylamino mit 1 bis 8 Kohlenstoffatomen im Alkylrest, Phenylamino, C₂-C₈-Alkoxycarbonyl, Nitro, Cyan, Trifluormethyl, Halogen, Sulfamoyl, am Stickstoffatom ein- oder zweifach durch C₁-C₄-Alkyl, C₅-C₇-Cycloalkyl oder Phenyl substituiertes Sulfamoyl, Carbamoyl, Ureido, Hydroxy, C₁-C₈-Alkylsulfonyl, Phenylsulfonyl, im Phenylring durch C₁-C₄-Alkyl, Halogen, Sulfo oder Sulfato substituiertes Phenylsulfonyl, Carboxy, Sulfomethyl, Sulfo, Sulfato, Thiosulfato und Phenylazo- und Naphthylazogruppen, und gegebenenfalls je 2 benachbarte Substituenten der genannten Ringsysteme weitere ankondensierte Phenylringe oder Cyclohexylringe bilden.

5. Azofarbstoffe gemäss einem der Ansprüche 1 bis 3, worin D Thiophenyl, Benzthiophenyl, Benzisothiazolyl, 1,3,4-Thiadiazolyl, 7-Oxotetrahydrobenzo[b]thiophenyl oder Phenyl ist, wobei jeder der genannten Reste substituiert sein kann durch C₁-C₈-Alkyl, C₁-C₈-Alkoxy, C₂-C₈-Alkanoyl, Carbamoyl, C₂-C₈-Alkoxycarbonyl, C₅-C₇-Cycloalkylcarbonyl, Benzoyl, im Phenylring durch C₁-C₄-Alkyl, Halogen, Sulfo oder Sulfato substituiertes Benzoyl, C₁-C₈-Alkylthio, durch Sulfo substituiertes C₁-C₈-Alkylthio, Benzthiazol, Benzoxazol, durch C₁-C₄-Alkyl, Halogen, Sulfo oder Sulfato substituiertes Benzthiazol oder Benzoxazol, Halogen, Sulfo, Trifluormethyl, Phenylsulfonyl, Naphthylsulfonyl, im Phenylring bzw. Naphthylring durch C₁-C₄-Alkyl, Halogen, Sulfo oder Sulfato substituiertes Phenylsulfonyl oder Naphthylsulfonyl, C₁-C₈-Alkylsulfonyl, 1-Azacycloheptan-N-sulfonyl, Sulfamoyl, am Stickstoffatom ein- oder zweifach durch C₁-C₄-Alkyl, C₅-C₇- Cycloalkyl, Phenyl oder Naphthyl substituiertes Sulfamoyl, im Phenyl- bzw. Naphthylring durch C₁-C₄-Alkyl, Halogen, Sulfo oder Sulfato substituiertes Phenyl- oder Naphthylaminosulfonyl, Phenoxy, Phenoxysulfonyl, durch C₁-C₄-Alkyl, Halogen, Sulfo oder Sulfato substituiertes Phenoxy oder Phenoxysulfonyl.

6. Azofarbstoffe gemäss einem der Ansprüche 1 bis 3, worin D Thiophenyl, 7-Oxotetrahydrobenzo[b]thiophenyl oder Phenyl ist, wobei jeder der genannten Reste substituiert sein kann durch C₁-C₈-Alkyl, C₂-C₈-Alkanoyl, C₂-C₈-Alkoxycarbonyl, C₅-C₇-Cycloalkylcarbonyl, Benzoyl, im Phenylring durch C₁-C₄-Alkyl, Halogen, Sulfo oder Sulfato substituiertes Benzoyl, Benzthiazol, Benzoxazol, durch C₁-C₄-Alkyl, Halogen, Sulfo oder Sulfato substituiertes Benzthiazol oder Benzoxazol, Halogen, Phenylsulfonyl, im Phenylring durch C₁-C₄-Alkyl, Halogen, Sulfo oder Sulfato substituiertes Phenylsulfonyl, C₁-C₈-Alkylsulfonyl, am Stickstoffatom ein- oder zweifach durch C₁-C₄-Alkyl, C₅-C₇-Cycloalkyl oder Phenyl substituiertes Sulfamoyl.

7. Azofarbstoffe gemäss einem der Ansprüche 1 bis 6, worin R₁ Wasserstoff oder C₁-C₄-Alkyl, insbesondere Methyl, ist.

8. Azofarbstoffe gemäss einem der Ansprüche 1 bis 7, worin R₂ und R₃ unabhängig voneinander Wasserstoff oder C₁-C₄-Alkyl, insbesondere Methyl, sind.

9. Azofarbstoffe gemäss einem der Ansprüche 1 bis 8, worin R₄ C₁-C₄-Alkyl, insbesondere Aethyl, oder Benzyl ist.

10. Azofarbstoffe gemäss einem der Ansprüche 1 bis 9, worin n die Zahl 1,2 oder 3, insbesondere 1 oder 2, ist.

11. Azofarbstoffe gemäss einem der Ansprüche 1 bis 6, worin R₁ Wasserstoff oder C₁-C₄-Alkyl, insbesondere Methyl, R₂ und R₃ unabhängig voneinander Wasserstoff oder C₁-C₄-Alkyl, insbesondere Methyl, R₄ C₁-C₄-Alkyl, insbesondere Aethyl, und n die Zahl 1 oder 2 ist.

12. Azofarbstoffe gemäss einem der Ansprüche 1 bis 11 der Formel 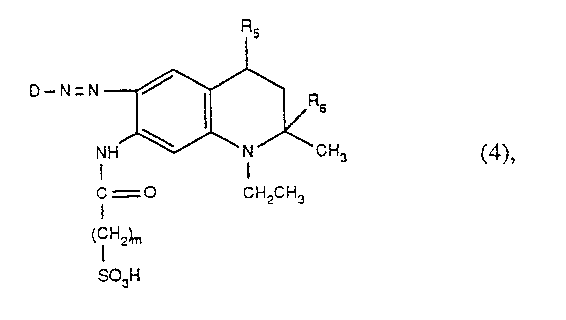 worin R₅ und R₆ unabhängig voneinander Wasserstoff oder Methyl und m die Zahl 1 oder 2 ist.

13. Azofarbstoffe gemäss Anspruch 12, worin R₅ und R₆ Wasserstoff oder R₅ und R₆ Methyl bedeuten.

14. Azofarbstoffe gemäss einem der Ansprüche 1 bis 13, welche nur zwei Sulfogruppen enthalten.

15. Verfahren zur Herstellung von Azofarbstoffen gemäss Anspruch 1, dadurch gekennzeichnet, dass man ein Amin der Formel
D-NH₂ (5)
diazotiert und auf eine Kupplungskomponente der Formel 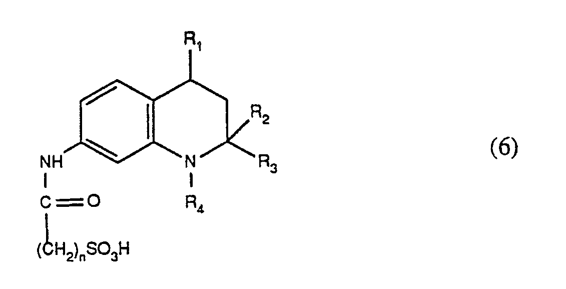 kuppelt, wobei D, R₁, R₂, R₃, R₄ und n die in Anspruch 1 angegebenen Bedeutungen haben.

16. Verwendung der Azofarbstoffe gemäss den Ansprüchen 1 bis 14, bzw. der gemäss Anspruch 15 erhaltenen Azofarbstoffe zum Färben und Bedrucken von stickstoffhaltigen oder hydroxygruppenhaltigen Fasermaterialien.

17. Verbindungen der Formel 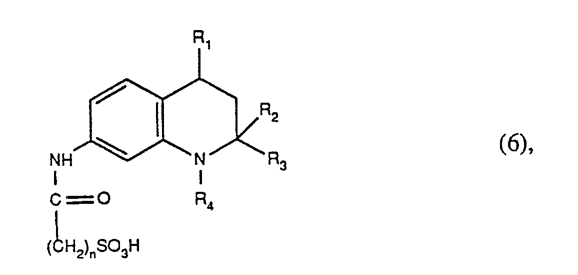 worin R₁, R₂, R₃ und R₄ unabhängig voneinander Wasserstoff oder gegebenenfalls substituiertes C₁-C₈-Alkyl und n die Zahl 1, 2, 3, 4 oder 5 ist.
